# EUROPEAN PATENT APPLICATION

(11) **EP 1 950 194 A1**
(43) Date of publication of application: **30.07.2008**
(21) Application number: 06823298.2
(22) Date of filing: 10.11.2006
(51) Int. Cl.: C07C 211/54, C07C 211/58, C09K 11/06, H01L 51/50

(54) **AROMATIC AMINE DERIVATIVE AND ORGANIC ELECTROLUMINESCENT ELEMENT USING THE SAME**

(30) Priority: 16.11.2005 JP 2005331382; 30.01.2006 JP 2006021003; 19.07.2006 US 458541
(71) Applicant: Idemitsu Kosan Co., Ltd., Chiyoda-ku Tokyo 100-8321 (JP)
(72) Inventor: YABUNOUCHI, Nobuhiro, Sodegaura-shi, Chiba 299-0293 (JP); KAWAMURA, Masahiro, Sodegaura-shi, Chiba 299-0293 (JP); YAMAMOTO, Hiroshi, Sodegaura-shi, Chiba 299-0293 (JP)
(74) Representative: Gille Hrabal Struck Neidlein Prop Roos
(86) International application number: PCT/JP2006/322476
(87) International publication number: WO 2007/058127

(57) **Abstract**

Provided are: a novel aromatic amine derivative having an asymmetric structure; and an organic electroluminescence device having one or multiple organic thin film layers including at least a light emitting layer, the one or multiple organic thin film layers being interposed between a cathode and an anode. The aromatic amine derivative realizes the organic EL device capable of suppressing the crystallization of a molecule, improving yields upon production of the organic EL device, and having a long lifetime when at least one layer of the one or more multiple organic thin film layers contains the aromatic amine derivative alone or as a component of a mixture.

## Description

### TECHNICAL FIELD

The present invention relates to aromatic amine derivatives and an organic electroluminescence (EL) device using the same, in particular, an aromatic amine derivative realizing the organic EL device capable of suppressing the crystallization of a molecule, improving yields upon production of the organic EL device, and of increasing the lifetime of the organic EL device by using an aromatic amine derivative having a specific substituent as a hole transporting material.

### BACKGROUND ART

An organic electroluminescence device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang et al. of Eastman Kodak Company (C. W. Tang and S. A. Vanslyke, Applied Physics Letters, Volume 51, Pages 913, 1987 or the like), many studies have been conducted on organic EL devices using organic materials as the composing materials. Tang et al. used tris(8-quinolinolato)aluminum for a light emitting layer and a triphenyldiamine derivative for a hole transporting layer. Advantages of the laminate structure are that the efficiency of hole injection into the light emitting layer can be increased, that the efficiency of forming exciton which are formed by blocking and recombining electrons injected from the cathode can be increased, and that exciton formed within the light emitting layer can be enclosed. As described above, for the constitution of the organic EL device, a two-layered structure having a hole transporting (injecting) layer and an electron-transporting and light emitting layer and a three-layered structure having a hole transporting (injecting) layer, a light emitting layer and an electron-transporting (injecting) layer are well known. To increase the efficiency of recombination of injected holes and electrons in the devices of the laminate type, the constitution of the device and the process for forming the device have been studied.

In general, when an organic EL device is driven or stored in an environment of a high temperature, adverse effects such as a change in the luminescent color, a decrease in emission efficiency, an increase in the voltage for driving, and a decrease in the lifetime of light emission arise. To prevent the adverse effects, it has been necessary that the glass transition temperature (Tg) of the hole transporting material be elevated. Therefore, it is necessary that the many aromatic groups be held within the molecule of the hole transporting material, and for example, the aromatic diamine derivative in Patent Document 1 and the fused aromatic ring diamine derivative in Patent Document 2, in general, a structure having 8 to 12 benzene rings may preferably be used.
However, when a large number of aromatic groups are present in a molecule, crystallization is apt to occur upon production of an organic EL device through the formation of a thin film by using those hole transporting materials. As a result, there arises a problem such as the clogging of the outlet of a crucible to be used in vapor deposition or a reduction in yields of the organic EL device due to the generation of a fault of the thin film resulting from the crystallization. In addition, a compound having a large number of aromatic groups in its molecules generally has a high glass transition temperature (Tg), but has a high sublimation temperature. Accordingly, there arises a problem in that the lifetime of the compound is short because a phenomenon such as decomposition at the time of vapor deposition or the formation of a nonuniform deposition film is expected to occur.
Meanwhile, there is a known document disclosing an asymmetric aromatic amine derivative. For example, Patent Document 3 describes an aromatic amine derivative having an asymmetric structure. However, the document has no specific example, and has no description concerning characteristics of an asymmetric compound. Further, the document has no description concerning characteristics in a specific unit shown in a general formula (2), and has no example of a blue device using a compound having the specific unit shown in the general formula (2). In addition, Patent Document 4 describes an asymmetric aromatic amine derivative having phenanthrene as an example. However, the derivative is treated in the same way as that of a symmetric compound, and the document has no description concerning characteristics of an asymmetric compound. In addition, none of those patents explicitly describes a method of producing an asymmetric compound in spite of the fact that the asymmetric compound requires a special synthesis method. Further, Patent Document 5 describes a method of producing an aromatic amine derivative having an asymmetric structure, but has no description concerning characteristics of an asymmetric compound. Patent Document 6 describes an asymmetric compound which has a high glass transition temperature and which is thermally stable, but exemplifies only a compound having carbazole. In addition, the inventors of the present invention have produced a device by using the compound. As a result, they have found that a problem lies in the short lifetime of the device.
As described above, an organic EL device having a long lifetime has been reported, but it cannot be said yet that the device always shows sufficient performance. In view of the foregoing, the development of an organic EL device having further excellent performance has been strongly desired.
[Patent Document 1] US 4,720,432
[Patent Document 2] US 5,061,569
[Patent Document 3] JP-A 08-48656
[Patent Document 4] JP-A 11-135261
[Patent Document 5] JP-A 2003-171366
[Patent Document 6] US 6,242,115

### DISCLOSURE OF THE INVENTION

### PROBLEMS TO BE SOLVED BY THE INVENTION

The present invention has been made with a view to solving the above-mentioned problems, and an object of the present invention is to provide an organic EL device in which a molecule hardly crystallizes, which can be produced with improved yields, and which has a long lifetime, and an aromatic amine derivative realizing the organic EL device.

### MEANS FOR SOLVING THE PROBLEMS

The inventors of the present invention have made extensive studies with a view toward achieving the above-mentioned object. As a result, they have found that the above-mentioned problems can be solved by using a novel aromatic amine derivative having a specific substituent represented by the following general formula (1) as a material for an organic EL device, in particular, a hole transporting material, thereby completing the present invention.
In addition, the inventors have found that an amino group substituted by an aryl group represented by a general formula (2) is suitable as an amine unit having a specific substituent. An interaction between molecules in the amine unit is small because the unit has steric hindrance. Accordingly, the unit has an effect in which: crystallization is suppressed; yield in which an organic EL device is produced is improved; and the lifetime of the resultant organic EL device is lengthened. It has been found that a significant lengthening effect on the lifetime can be obtained by combining the unit with, in particular, a blue light emitting device.

The present invention provides an aromatic amine derivative represented by the following general formula (1): 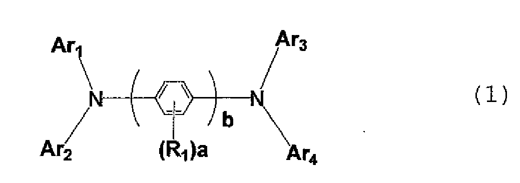 where:
R₁ represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxygroup having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxyl group, or a carboxyl group;
a represents an integer of 0 to 4, b represents an integer of 1 to 3, and, when b represents 2 or more, multiple R₁s may be bonded to each other to forma saturated or unsaturated, five-membered or six-membered cyclic structure which may be substituted; and
at least one of Ar₁ to Ar₄ represents a group of the following general formula (2): 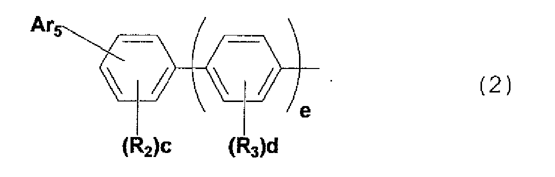
where:
R₂ and R₃ are each independently selected from the same groups as those of R₁ in the general formula (1);
Ar₅ represents a fused aromatic ring group having 6 to 20 ring carbon atoms;
c and d each represent an integer of 0 to 4, and e represents an integer of 0 to 2;
R₂ and R₃, or multiple R₃s may be bonded to each other to form a saturated or unsaturated, five-membered or six-membered cyclic structure which may be substituted; and
groups among Ar₁ to Ar₄ none of which is represented by the general formula (2) each independently represent a substituted or unsubstituted aryl group having 6 to 50 ring carbon atoms, or a substituted or unsubstituted aromatic heterocyclic group having 5 to 50 ring carbon atoms.

Further, the present invention provides an organic electroluminescence device, including one or multiple organic thin film layers including at least a light emitting layer, the one or multiple organic thin film layers being interposed between a cathode and an anode, in which at least one layer of the one or more multiple organic thin film layers contains the aromatic amine derivative alone or as a component of a mixture.

### EFFECT OF THE INVENTION

An organic EL device using the aromatic amine derivative of the present invention hardly causes the crystallization of molecules, can be produced with improved yields, and has a long lifetime.

### BEST MODE FOR CARRYING OUT THE INVENTION

An aromatic amine derivative of the present invention is represented by the following general formula (1). 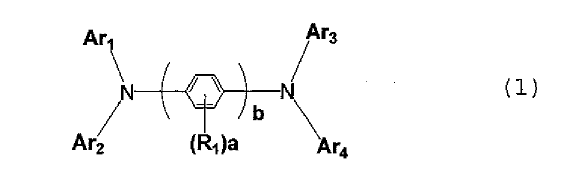 In the general formula (1), R₁ represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxyl group, or a carboxyl group.
In the general formula (1), a represents an integer of 0 to 4, and b represents an integer of 1 to 3. When b represents 2 or more, multiple R₁s may be bonded to each other to form a saturated or unsaturated, five-membered or six-membered cyclic structure which may be substituted.
At least one of Ar₁ to Ar₄ represents a group of the following general formula (2). 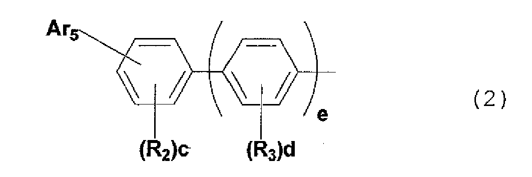 In the general formula (2), R₂ and R₃ are each independently selected from the same groups as those of R₁ in the general formula (1). Ar₅ represents a fused aromatic ring group having 6 to 20 ring carbon atoms. c and d each represent an integer of 0 to 4, and e represents an integer of 0 to 2. R₂ and R₃, or multiple R₃s may be bonded to each other to form a saturated or unsaturated, five-membered or six-membered cyclic structure which may be substituted.
In the general formula (1), groups among Ar₁ to Ar₄ none of which is represented by the general formula (2) each independently represent a substituted or unsubstituted aryl group having 6 to 50 ring carbon atoms, or a substituted or unsubstituted aromatic heterocyclic group having 5 to 50 ring carbon atoms.
The aromatic amine derivative of the general formula (1) of the present invention has a total number of carbon atoms except those of a substituent of 56 or more, preferably 58 or more, or more preferably 68 to 80.

Examples of the aryl group of any one of R₁ to R₃ in the general formulae (1) and (2) include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-yl group, anm-terphenyl-2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, a fluoranthenyl group, a fluorenyl group, a 1-pyrolyl group, a 2-pyrolyl group, a 3-pyrolyl group, a pyradinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrolin-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1,10-phenanthrolin-3-yl group, a 1,10-phenanthrolin-4-yl group, a 1,10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a 1-phenadinyl group, a 2-phenadinyl group, a 1-phenothiadinyl group, a 2-phenothiadinyl group, a 3-phenothiadinyl group, a 4-phenothiadinyl group, a 10-phenothiadinyl group, a 1-phenoxadinyl group, a 2-phenoxadinyl group, a 3-phenoxadinyl group, a 4-phenoxadinyl group, a 10-phenoxadinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrol-1-yl group, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group, and a 4-t-butyl-3-indolyl group.
Of those, a phenyl group, a naphthyl group, a biphenyl group, an anthranyl group, a phenanthryl group, a pyrenyl group, a crycenyl group, a fluoranthenyl group, and a fluorenyl group are preferable.

Examples of the alkyl group of any one of R₁ to R₃ in the general formulae (1) and (2) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1, 2, 3-tribromopropyl group, aniodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

The alkoxy group of any one of R₁ to R₃ in the general formulae (1) and (2) is represented by -OY, and examples of Y include the same examples as those described for the above-mentioned alkyl group.
Examples of the aralkyl group of any one of R₁ to R₃ in the general formulae (1) and (2) include a benzyl group, a 1-phenylethyl group, a 2-phenylethyl group; a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, an m-methylbenzyl group, an o-methylbenzyl group, a p-chlorobenzyl group, an m-chlorobenzyl group, an o-chlorobenzyl group, a p-bromobenzyl group, anm-bromobenzyl group, an o-bromobenzyl group, a p-iodobenzyl group, an m-iodobenzyl group, an o-iodobenzyl group, a p-hydroxybenzyl group, an m-hydroxybenzyl group, an o-hydroxybenzyl group, a p-aminobenzyl group, an m-aminobenzyl group, an o-aminobenzyl group, a p-nitrobenzyl group, an m-nitrobenzyl group, an o-nitrobenzyl group, a p-cyanobenzyl group, an m-cyanobenzyl group, an o-cyanobenzyl group, a 1-hydroxy-2-phenylisopropyl group, and a 1-chloro-2-phenylisopropyl group.

The aryloxy group of any one of R₁ to R₃ in the general formulae (1) and (2) is represented by -OY', and examples of Y' include the same examples as those described for the above-mentioned aryl group.
The arylthio group of any one of R₁ to R₃ in the general formulae (1) and (2) is represented by -SY', and examples of Y' include the same examples as those described for the above-mentioned aryl group.
The alkoxycarbonyl group of any one of R₁ to R₃ in the general formulae (1) and (2) is a group represented by -COOY, and examples of Y include the same examples as those described for the above-mentioned alkyl group.
Examples of an aryl group in the amino group substituted by the aryl group of any one of R₁ to R₃ in the general formulae (1) and (2) include the same examples as those described for the above-mentioned aryl group.
Examples of the halogen atom of any one of R₁ to R₃ in the general formulae (1) and (2) include a fluorine atom, a chlorine atom, a bromine atom, and an iodine atom.

In the general formula (1), a represents an integer of 0 to 4, b represents an integer of 1 to 3, and, when b represents 2 or more, multiple R₁'s may be bonded to each other to form a saturated or unsaturated, five-membered or six-membered cyclic structure which may be substituted. In addition, in the general formula (2), R₂ and R₃, or multiple R₃'s may be bonded to each other to form a saturated or unsaturated, five-membered or six-membered cyclic structure which may be substituted.
Examples of the five-membered or six-membered cyclic structure which may be formed include: cycloalkanes each having 4 to 12 carbon atoms such as cyclopentane, cyclohexane, adamantane, and norbornane; cycloalkenes each having 4 to 12 carbon atoms such as cyclopentene and cyclohexene; cycloalkadienes each having 6 to 12 carbon atoms such as cyclopentadiene and cyclohexadiene; and aromatic rings each having 6 to 50 carbon atoms such as benzene, naphthalene, phenanthrene, anthracene, pyrene, chrysene, and acenaphthylene.

In the aromatic amine derivative of the present invention, Ar₁ and Ar₂ in the general formula (1) are each preferably represented by the general formula (2).
In the aromatic amine derivative of the present invention, Ar₁ and Ar₃ in the general formula (1) are each preferably represented by the general formula (2).
In the aromatic amine derivative of the present invention, e in the general formula (2) preferably represents 0.

Examples of the fused aromatic ring as Ar₅ in the general formula (2) include a 1-naphthyl group, a 2-naphthyl group, a phenanthryl group, and a pyrenyl group. Of those, a 1-naphthyl group or a 2-naphthyl group is preferable.

In the aromaticamine derivative of the present invention, Ar₂ in the general formula (1) is preferably represented by the following general formula (3). 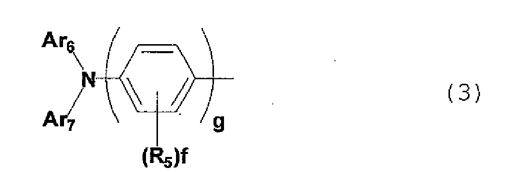 In the general formula (3) : R₅ is selected from the same groups as those of R₁ in the general formula (1); f represents an integer of 0 to 4, and g represents an integer of 1 to 3; when g represents 2 or more, multiple R₅'s may be bonded to each other to form a saturated or unsaturated, five-membered or six-membered cyclic structure which may be substituted; and Ar₆ and Ar₇ are each represented by the general formula (2), or each independently represent a substituted or unsubstituted aryl group having 6 to 50 ring carbon atoms, or a substituted or unsubstituted aromatic heterocyclic group having 5 to 50 ring carbon atoms.
Examples of the respective substituents of R₅ are the same as those exemplified for R₁ to R₃ in the general formulae (1) and (2). Examples of the five-membered or six-membered cyclic structure of R₅ are the same as those exemplified for the general formulae (1) and (2).
Further, a substituent for any one of Ar₁ to Ar₇ is a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxyl group, or a carboxyl group. In addition, specific examples of the alkyl group, alkoxy group, aralkyl group, aryloxy group, arylthio group, alkoxycarbonyl group, and amino group substituted by an aryl group for any one of Ar₁ to Ar₇ include the same examples as those described for R₁ to R₃.

In the aromatic amine derivative of the present invention, Ar₂ and Ar₄ in the general formula (1) are preferably each independently represented by the general formula (3).

The aromatic amine derivative of the present invention is preferably a material for an organic electroluminescence device.
The aromatic amine derivative of the present invention is preferably a hole transporting material for an organic electroluminescence device.

An organic electroluminescence device of the present invention is preferably an organic electroluminescence device including one or multiple organic thin film layers including at least a light emitting layer, the one or multiple organic thin film layers being interposed between a cathode and an anode, in which at least one layer of the one or more multiple organic thin film layers contains the aromatic amine derivative of the present invention alone or as a component of a mixture.
In the organic electroluminescence device of the present invention, the aromatic amine derivative of the present invention is preferably incorporated into a hole transporting layer.
The organic electroluminescence device of the present invention preferably emits bluish light.
In the organic electroluminescence device of the present invention, the light emitting layer preferably contains styrylamine and/or arylamine.

Specific examples of the aromatic amine derivative represented by the general formula (1) of the present invention are shown below. However, the derivative is not limited to these exemplified compounds.

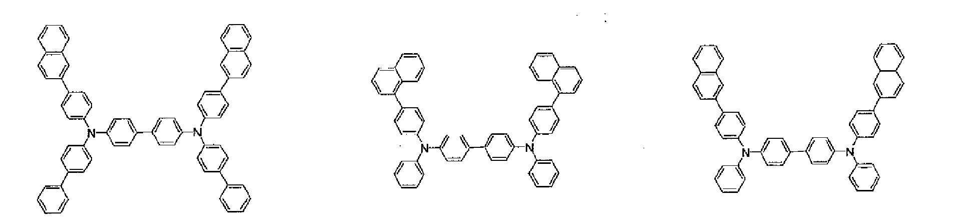

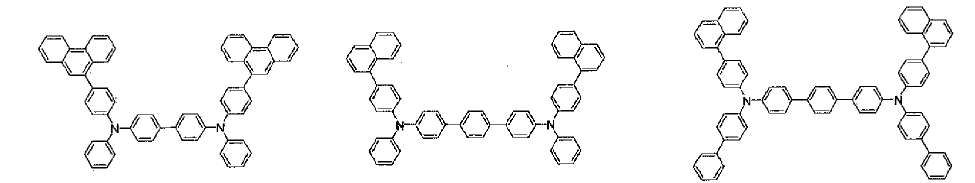

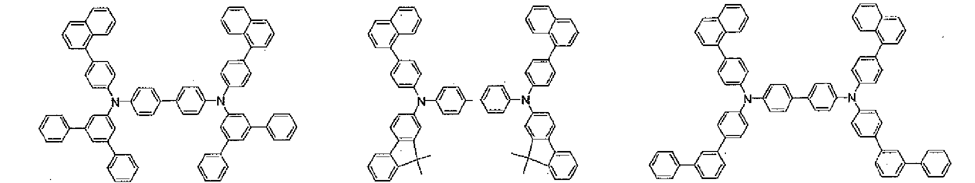

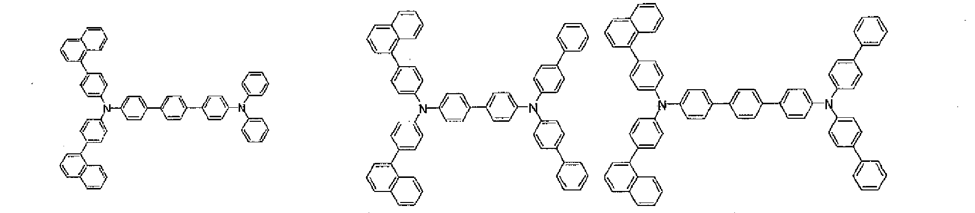

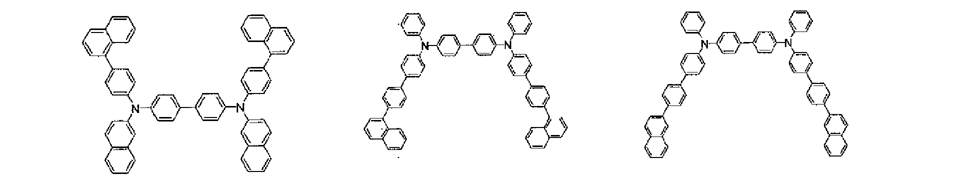

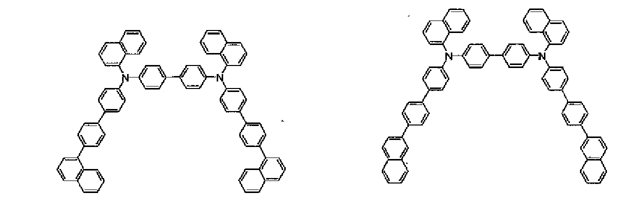

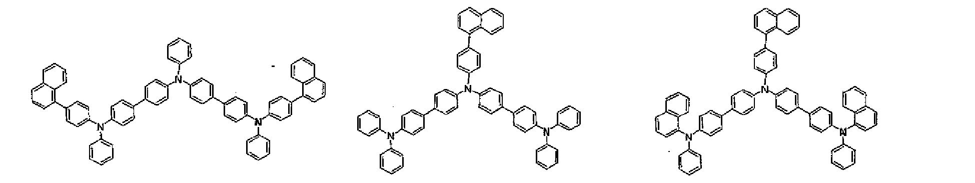

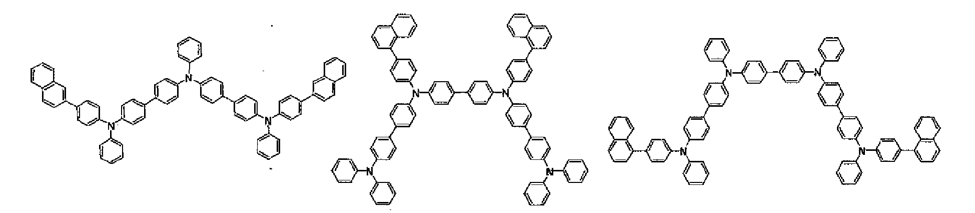

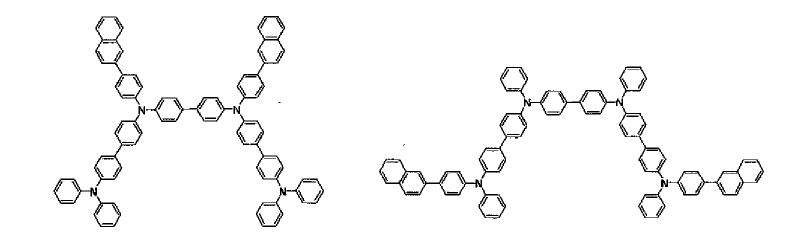

Next, the organic EL device of the present invention will be described.
An organic EL device of the present invention includes one or multiple organic thin film layers including at least a light emitting layer, the one or multiple organic thin film layers being interposed between a cathode and an anode, in which at least one layer of the one or more multiple organic thin film layers contains the aromatic amine derivative alone or as a component of a mixture.
In the organic EL device of the present invention, it is preferable that: the one or multiple organic thin film layers have a hole transporting layer; and the hole transporting layer contain the aromatic amine derivative of the present invention alone or as a component of a mixture. It is more preferable that the hole transporting layer contain the aromatic amine derivative of the present invention as a main component.
The aromatic amine derivative of the present invention is particularly preferably used in an organic EL device that emits bluish light.

In addition, in the organic EL device of the present invention, the light emitting layer preferably contains an aryl amine compound and/or a styrylamine compound.
Examples of the arylamine compound include compounds each represented by the following general formula (I), and examples of the styrylamine compound include compounds each represented by the following general formula (II):

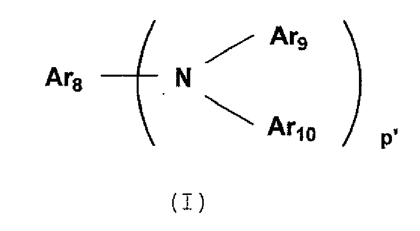

[In the general formula (I), Ar₈ represents a group selected from phenyl, biphenyl, terphenyl, stilbene, and distyrylaryl groups, Ar₉ and Ar₁₀ each represent a hydrogen atom or an aromatic group having 6 to 20 carbon atoms, each of Ar₉ and Ar₁₀ may be substituted, p' represents an integer of 1 to 4, and Ar₉ and/or Ar₁₀ are/is more preferably substituted by styryl groups/a styryl group.]
Here, the aromatic group having 6 to 20 carbon atoms is preferably a phenyl group, a naphthyl group, an anthranyl group, a phenanthryl group, a terphenyl group, or the like.

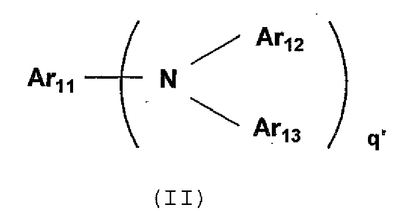

[In the general formula (II), Ar₁₁ to Ar₁₃ each represent an aryl group which has 5 to 40 ring carbon atoms and which may be substituted, and q' represents an integer of 1 to 4.]
Here, examples of the aryl group having 5 to 40 ring atoms preferably include phenyl, naphthyl, anthranyl, phenanthryl, pyrenyl, coronyl, biphenyl, terphenyl, pyrrolyl, furanyl, thiophenyl, benzothiophenyl, oxadiazolyl, diphenylanthranyl, indolyl, carbazolyl, pyridyl, benzoquinolyl, fluoranthenyl, acenaphthofluoranthenyl, and stylbene. In addition, the aryl group having 5 to 40 ring atoms may further be substituted by a substituent. Examples of the substituent preferably include: an alkyl group having 1 to 6 carbon atoms such as an ethyl group, a methyl group, an i-propyl group, an n-propyl group, an s-butyl group, a t-butyl group, a pentyl group, a hexyl group, a cyclopentyl group, or a cyclohexyl group; an alkoxy group having 1 to 6 carbon atoms such as an ethoxy group, a methoxy group, an i-propoxy group, an n-propoxy group, an s-butoxy group, a t-butoxy group, a pentoxy group, a hexyloxy group, a cyclopentoxy group, or a cyclohexyloxy group; an aryl group having 5 to 40 ring atoms; an amino group substituted by an aryl group having 5 to 40 ring atoms; an ester group containing an aryl group having 5 to 40 ring atoms; an ester group containing an alkyl group having 1 to 6 carbon atoms; a cyano group; a nitro group; and a halogen atom such as chlorine, bromine, or iodine.

The constitution of the organic EL device of the present invention will be described in the following.

### (1) Organic EL device constitution

TTypical examples of the constitution of the organic EL device of the present invention include the following:
(1) an anode/light emitting layer/cathode;
(2) an anode/hole injecting layer/light emitting layer/cathode;
(3) an anode/light emitting layer/electron injecting layer/cathode;
(4) an anode/hole injecting layer/light emitting layer/electron injecting layer/cathode;
(5) an anode/organic semiconductor layer/light emitting layer/cathode;
(6) an anode/organic semiconductor layer/electron barrier layer/light emitting layer/cathode;
(7) an anode/organic semiconductor layer/light emitting layer/adhesion improving layer/cathode;
(8) an anode/hole injecting layer/hole transporting layer/light emitting layer/electron injecting layer/cathode;
(9) an anode/insulating layer/light emitting layer/insulating layer/cathode;
(10) an anode/inorganic semiconductor layer/insulating layer/light emitting layer/insulating layer/cathode;
(11) an anode/organic semiconductor layer/insulating layer/light emitting layer/insulating layer/cathode;
(12) an anode/insulating layer/hole injecting layer/hole transporting layer/light emitting layer/ insulating layer/cathode; and
(13) an anode/insulating layer/hole injecting layer/hole transporting layer/light emitting layer/electron injecting layer/cathode.
Of those, the constitution (8) is preferably used in ordinary cases. However, the constitution is not limited to the foregoing.
The aromatic amine derivative of the present invention may be used in any one of the organic thin film layers of the organic EL device. The derivative can be used in a light emitting band or a hole transporting band. The derivative is used preferably in the hole transporting band, or particularly preferably in a hole transporting layer, thereby making a molecule hardly crystallize and improving yields upon production of the organic EL device.
The amount of the aromatic amine derivative of the present invention to be incorporated into the organic thin film layers is preferably 30 to 100 mol%.

### (2) Transparent substrate

The organic EL device of the present invention is prepared on a transparent substrate. Here, the transparent substrate is the substrate which supports the organic EL device. It is preferable that the transparent substrate have a transmittance of light of 50% or greater in the visible region of 400 to 700 nm and be flat and smooth.
Examples of the transparent substrate include glass plates and polymer plates. Specific examples of the glass plate include plates made of soda-lime glass, glass containing barium and strontium, lead glass, aluminosilicate glass, borosilicate glass, barium borosilicate glass, and quartz. Specific examples of the polymer plate include plates made of polycarbonate, polyacrylate, polyethylene terephthalate, polyether sulfide, and polysulfone.

### (3) Anode

The anode in the organic EL device of the present invention has the function of injecting holes into the hole transporting layer or the light emitting layer. It is effective that the anode has a work function of 4.5 eV or greater. Specific examples of the material for the anode used in the present invention include indium tin oxide alloys (ITO), tin oxide (NESA), indium zinc oxide (IZO), gold, silver, platinum, and copper.
The anode can be prepared by forming a thin film of the electrode material described above in accordance with a process such as the vapor deposition process and the sputtering process.
When the light emitted from the light emitting layer is obtained through the anode, it is preferable that the anode have a transmittance of the emitted light greater than 10%. It is also preferable that the sheet resistivity of the anode be several hundred Ω/□ or smaller. The thickness of the anode is, in general, selected in the range of 10 nm to 1 µm and preferably in the range of 10 to 200 nm although the preferable range may be different depending on the used material.

### (4) Light emitting layer

The light emitting layer in the organic EL device has a combination of the following functions (1) to (3).
(1) The injecting function: the function of injecting holes from the anode or the hole injecting layer and injecting electrons from the cathode or the electron injecting layer when an electric field is applied.
(2) The transporting function: the function of transporting injected charges (i.e., electrons and holes) by the force of the electric field.
(3) The light emitting function: the function of providing the field for recombination of electrons and holes and leading to the emission of light.
   However, the easiness of injection may be different between holes and electrons and the ability of transportation expressed by the mobility may be different between holes and electrons. It is preferable that either one of the charges be transferred.
   For the process for forming the light emitting layer, a known process such as the vapor deposition process, the spin coating process, and the LB process can be used. It is particularly preferable that the light emitting layer be a molecular deposit film. The molecular deposit film is a thin film formed by deposition of a material compound in the gas phase or a film formed by solidification of a material compound in a solution or in the liquid phase. In general, the molecular deposit film can be distinguished from the thin film formed in accordance with the LB process (i.e., molecular accumulation film) based on the differences in aggregation structure and higher order structure and functional differences caused by those structural differences.
   Further, as disclosed in JP-A 57-51781, the light emitting layer can also be formed by dissolving a binder such as a resin and the material compounds into a solvent to prepare a solution, followed by forming a thin film from the prepared solution by the spin coating process or the like.
   In the present invention, where desired, the light emitting layer may include other known light emitting materials other than the light emitting material composed of the aromatic amine derivative of the present invention, or a light emitting layer including other known light emitting material may be laminated to the light emitting layer including the light emitting material composed of the aromatic amine derivative of the present invention as long as the object of the present invention is not adversely affected.

Examples of a light emitting material or a doping material which can be used in the light emitting layer together with the aromatic amine derivative of the present invention include, but not limited to, anthracene, naphthalene, phenanthrene, pyrene, tetracene, coronene, chrysene, fluoresceine, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, quinoline metal complexes, aminoquinoline metal complexes, benzoquinoline metal complexes, imine, diphenylethylene, vinylanthracene, diaminocarbazole, pyrane, thiopyrane, polymethine, merocyanine, imidazole-chelated oxynoid compounds, quinacridone, rubrene, and fluorescent dyes.

A host material that can be used in a light emitting layer together with the aromatic amine derivative of the present invention is preferably a compound represented by any one of the following formulae (i) to (ix).
An asymmetric anthracene represented by the following general formula (i): 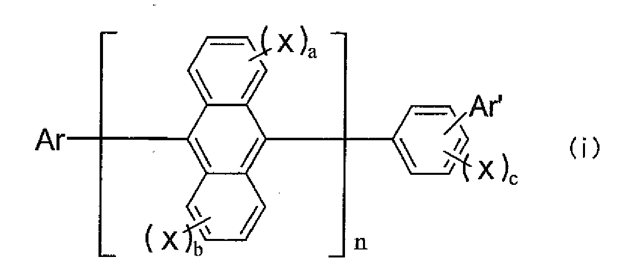 where:
Ar represents a substituted or unsubstituted fused aromatic group having 10 to 50 ring atoms;
Ar' represents a substituted or unsubstituted aromatic group having 6 to 50 ring carbon atoms;
X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group, or a hydroxyl group.
a, b, and c each represent an integer of 0 to 4; and
n represents an integer of 1 to 3. In addition, when n represents 2 or more, anthracene nuclei in [] may be identical to or different from each other.

An asymmetric monoanthracene derivative represented by the following general formula (ii): 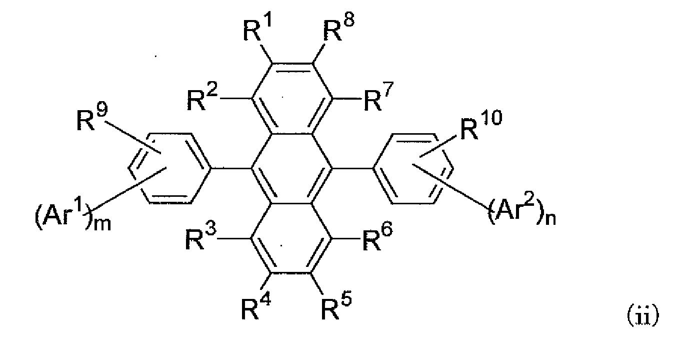 where:
Ar¹ and Ar² each independently represent a substituted or unsubstituted aromatic ring group having 6 to 50 ring carbon atoms. m and n each represent an integer of 1 to 4; provided that Ar¹ and Ar² are not identical to each other when m = n = 1 and positions at which Ar¹ and Ar² are bound to a benzene ring are bilaterally symmetric, and m and n represent different integers when m or n represents an integer of 2 to 4; and
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group.

An asymmetric pyrene derivative represented by the following general formula (iii): 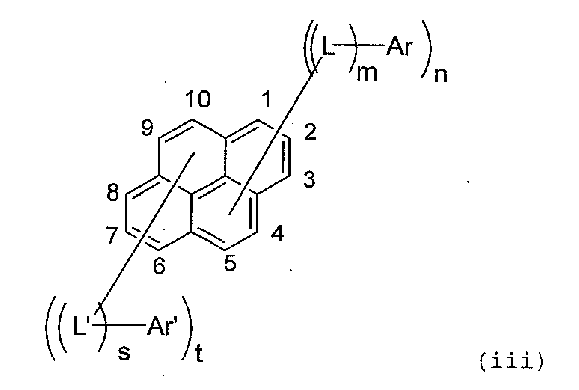 where:
Ar and Ar' each represent a substituted or unsubstituted aromatic group having 6 to 50 ring carbon atoms;
L and L' each represent a substituted or unsubstituted phenylene group, a substituted or unsubstituted naphthalenylene group, a substituted or unsubstituted fluorenylene group, or a substituted or unsubstituted dibenzosilolylene group;
m represents an integer of 0 to 2. n represents an integer of 1 to 4. s represents an integer of 0 to 2. t represents an integer of 0 to 4; and
   in addition, L or Ar binds to any one of 1- to 5-positions of pyrene, and L' or Ar' binds to any one of 6- to 10-positions of pyrene;
   provided that Ar, Ar', L, and L' satisfy the following item (1) or (2) when n + t represents an even number,
   (1) Ar ≠ Ar' and/or L ≠ L' (where the symbol "≠" means that groups connected with the symbol have different structures)
   (2) When Ar = Ar' and L = L',
      (2-1) m ≠ s and/or n ≠ t, or
      (2-2) when m = s and n = t,
         (2-2-1) L and L' (or pyrene) bind (or binds) to different binding positions on Ar and Ar', or
         (2-2-2) in the case where L and L' (or pyrene) bind (or binds) to the same binding positions on Ar and Ar', the case where the substitution positions of L and L', or of Ar and Ar' in pyrene are 1- and 6-positions, or 2- and 7-positions does not occur.

An asymmetric anthracene derivative represented by the following general formula (iv): 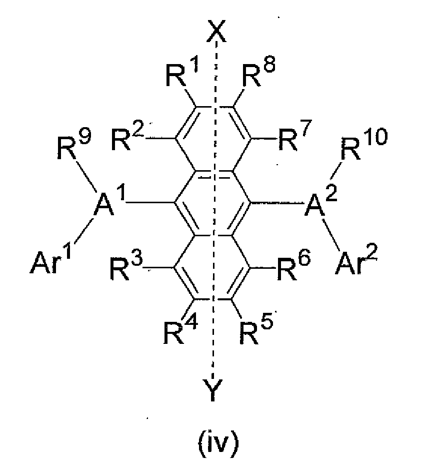 where:
A¹ and A² each independently represent a substituted or unsubstituted fused aromatic ring group having 10 to 20 ring carbon atoms;
Ar¹ and Ar² each independently represent a hydrogen atom, or a substituted or unsubstituted aromatic ring group having 6 to 50 ring carbon atoms;
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group; and
the number of each of Ar¹, Ar², R⁹, and R¹⁰ may be two or more, and adjacent groups may form a saturated or unsaturated cyclic structure;
provided that the case where groups symmetric with respect to the X-Y axis shown on central anthracene in the general formula (1) bind to 9- and 10-positions of the anthracene does not occur.

An anthracene derivative represented by the following general formula (v): 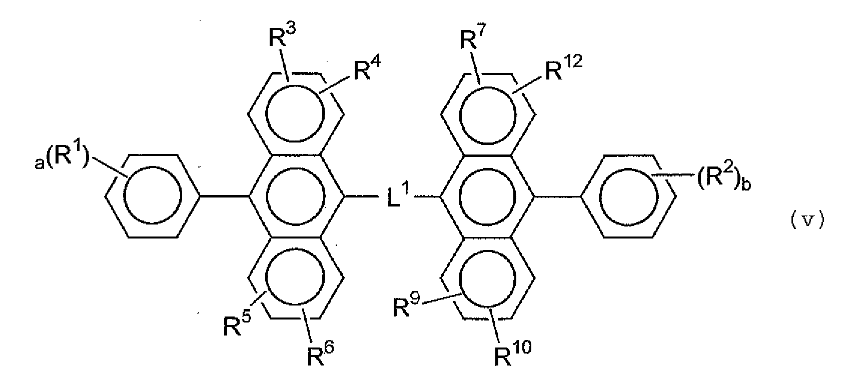 where: R¹ to R¹⁰ each independently represent a hydrogen atom, an alkyl group, a cycloalkyl group, an aryl group whichmaybe substituted, an alkoxyl group, an aryloxy group, an alkylamino group, an alkenyl group, an arylamino group, or a heterocyclic group which may be substituted; a and b each represent an integer of 1 to 5, and, when a or b represents 2 or more, R¹'s or R²'s may be identical to or different from each other, or R¹'s or R²'s may be bonded to each other to form a ring; R³ and R⁴, R⁵ and R⁶, R⁷ and R⁸, or R⁹ and R¹⁰ may be bonded to each other to form a ring; and L¹ represents a single bond, -O-, -S-, -N(R) - where R represents an alkyl group or an aryl group which may be substituted, an alkylene group, or an arylene group.

An anthracene derivative represented by the following general formula (vi): 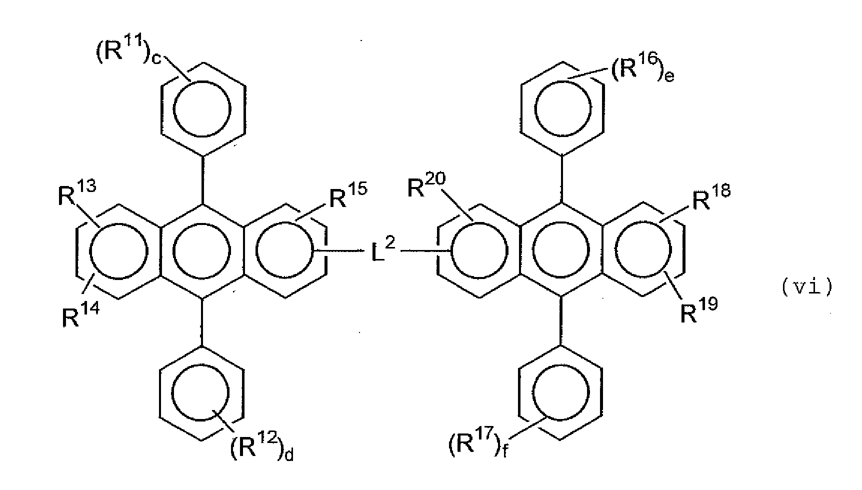 where: R¹¹ to R²⁰ each independently represent a hydrogen atom, an alkyl group, a cycloalkyl group, an aryl group, an alkoxyl group, an aryloxy group, an alkylamino group, an arylamino group, or a heterocyclic group which may be substituted; c, d, e, and f each represent an integer of 1 to 5, and, when any one of c, d, e, and f represents 2 or more, R¹¹'s, R¹²'s, R¹⁶'s, or R¹⁷'s may be identical to or different from each other, or R¹¹'s, R¹²'s, R¹⁶'s, or R¹⁷'s may be bonded to each other to form a ring; R¹³ and R¹⁴, or R¹⁸ and R¹⁹ may be bonded to each other to form a ring; and L² represents a single bond, -O-, -S-, -N(R)- where R represents an alkyl group or an aryl group which may be substituted, an alkylene group, or an arylene group.

A spirofluorene derivative represented by the following general formula (vii): 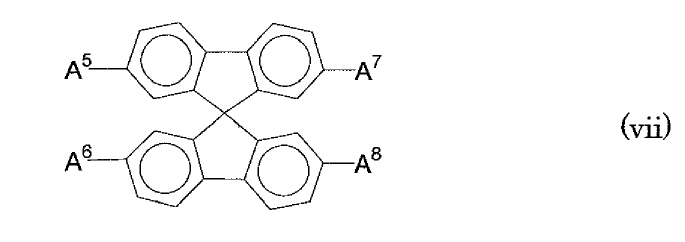 where A⁵ to A⁸ each independently represent a substituted or unsubstituted biphenyl group, or a substituted or unsubstituted naphthyl group.

A fused ring-containing compound represented by the following general formula (viii): 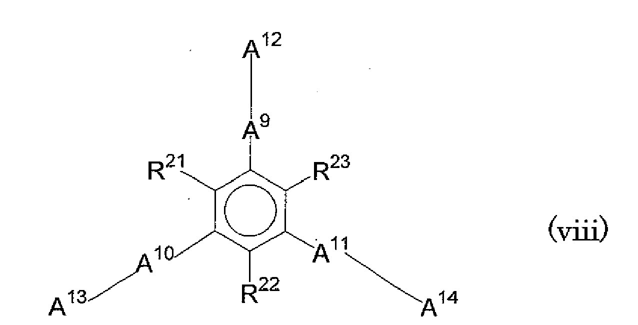 where: A⁹ to A¹⁴ each have the same meaning as that described above; R²¹ to R²³ each independently represent a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms, or a halogen atom; and at least one of A⁹ to A¹⁴ represents a group having three or more fused aromatic rings.

A fluorene compound represented by the following general formula (ix): 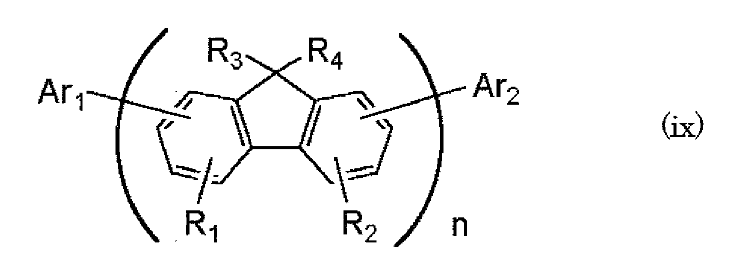 where: R₁ and R₂ each represent a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group, or a halogen atom; R₁'s or R₂'s bonded to different fluorene groups may be identical to or different from each other, and R₁ and R₂ bonded to the same fluorene group may be identical to or different from each other; R₃ and R₄ each represent a hydrogen atom, a substituted or unsubstituted alkyl group, a substituted or unsubstituted aralkyl group, a substituted or unsubstituted aryl group, or a substituted or unsubstituted heterocyclic group; R₃'s or R₄'s bonded to different fluorene groups may be identical to or different from each other, and R₃ and R₄ bonded to the same fluorene group may be identical to or different from each other; Ar₁ and Ar₂ each represent a substituted or unsubstituted fused polycyclic aromatic group having three or more benzene rings in total, or a substituted or unsubstituted fused polycyclic heterocyclic group that has three or more rings each of which is a benzene ring or a heterocyclic ring in total and that is bonded to a fluorene group by carbon, and Ar₁ and Ar₂ may be identical to or different from each other; and n represents an integer of 1 to 10.

Of the above-mentioned host materials, an anthracene derivative is preferable, a monoanthracene derivative is more preferable, and an asymmetric anthracene is particularly preferable.
In addition, a phosphorescent compound can also be used as a light emitting material for a dopant. A compound containing a carbazole ring as a host material is preferable as the phosphorescent compound. The dopant is a compound capable of emitting light from a triplet exciton, and is not particularly limited as long as light is emitted from a triplet exciton, a metal complex containing at least one metal selected from the group consisting of Ir, Ru, Pd, Pt, Os, and Re is preferable, and a porphyrin metal complex or an orthometalated metal complex is preferable.
A host composed of a compound containing a carbazole ring and suitable for phosphorescence is a compound having a function of causing a phosphorescent compound to emit light as a result of the occurrence of energy transfer from the excited state of the host to the phosphorescent compound. A host compound is not particularly limited as long as it is a compound capable of transferring exciton energy to a phosphorescent compound, and can be appropriately selected in accordance with a purpose. The host compound may have, for example, an arbitrary heterocyclic ring in addition to a carbazole ring.

Specific examples of such a host compound include polymer compounds such as a carbazole derivative, a triazole derivative, an oxazole derivative, an oxadiazole derivative, an imidazole derivative, a polyarylalkane derivative, a pyrazoline derivative, a pyrazolone derivative, a phenylene diamine derivative, an aryl amine derivative, an amino substituted chalcone derivative, a styrylanthracene derivative, a fluorenone derivative, a hydrazone derivative, a stylbene derivative, a silazane derivative, an aromatic tertiary amine compound, a styryl amine compound, an aromatic dimethylidene-based compound, a porphyrin-based compound, an anthraquinodimethane derivative, an anthrone derivative, a diphenylquinone derivative, a thiopyranedioxide derivative, a carbodiimide derivative, a fluorenilidene methane derivative, a distyryl pyrazine derivative, a heterocyclic tetracarboxylic anhydride such as naphthaleneperylene, a phthalocyanine derivative, various metal complex polysilane-based compounds typified by a metal complex of an 8-quinolinol derivative or a metal complex having metal phthalocyanine, benzooxazole, or benzothiazole as a ligand, a poly(N-vinylcarbazole) derivative, an aniline-based copolymer, a conductive high molecular weight oligomer such as a thiophene oligomer or polythiophene, a polythiophene derivative, a polyphenylene derivative, a polyphenylene vinylene derivative, and a polyfluorene derivative. One of the host materials may be used alone, or two or more of them may be used in combination.
Specific examples thereof include the compounds as described below.

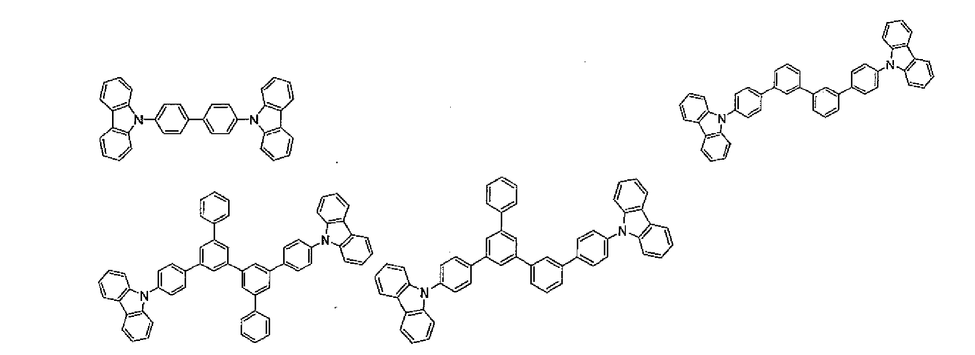

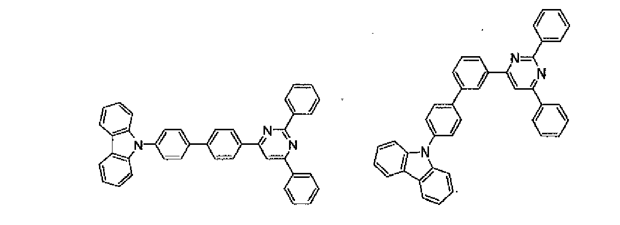

Aphosphorescent dopant is a compound capable of emitting light from a triplet exciton. The dopant, which is not particularly limited as long as light is emitted from a triplet exciton, is preferably a metal complex containing at least one metal selected from the group consisting of Ir, Ru, Pd, Pt, Os, and Re, and is preferably a porphyrin metal complex or an orthometalated metal complex. A porphyrin platinum complex is preferable as the porphyrin metal complex. One kind of a phosphorescent compound may be used alone, or two or more kinds of phosphorescent compounds may be used in combination.
Any one of various ligands can be used for forming an orthometalated metal complex. Examples of a preferable ligand include a 2-phenylpyridine derivative, a 7,8-benzoquinoline derivative, a 2-(2-thienyl)pyridine derivative, a 2-(1-naphthyl)pyridine derivative, and a 2-phenylquinoline derivative. Each of those derivatives may have a substituent as required. A fluoride or a group obtained by introducing a trifluoromethyl group is particularly preferable as bluish dopant. The metal complex may further include a ligand other than the above-mentioned ligands such as acetylacetonate or picric acid as an auxiliary ligand.
The content of the phosphorescent dopant in the light emitting layer is not particularly limited, and can be appropriately selected in accordance with a purpose. The content is, for example, 0.1 to 70 mass%, and is preferably 1 to 30 mass%. When the content of the phosphorescent compound is less than 0.1 mass%, the intensity of emitted light is weak, and an effect of the incorporation of the compound is not sufficiently exerted. When the content exceeds 70 mass%, a phenomenon referred to as concentration quenching becomes remarkable, and device performance reduces.
In addition, the light emitting layer may contain a hole transporting material, an electron transporting material, or a polymer binder as required.
Further, the thickness of the light emitting layer is preferably 5 to 50 nm, more preferably 7 to 50 nm, or most preferably 10 to 50 nm. When the thickness is less than 5 nm, it becomes difficult to form the light emitting layer, so the adjustment of chromaticity may be difficult. When the thickness exceeds 50 nm, the voltage at which the device is driven may increase.

(5) Hole injecting and transporting layer (hole transporting zone) The hole injecting and transporting layer is a layer which helps injection of holes into the light emitting layer and transports the holes to the light emitting region. The layer exhibits a great mobility of holes and, in general, has an ionization energy as small as 5.5 eV or smaller. For such the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.
When the aromatic amine derivative of the present invention is used in the hole transporting zone, the aromatic amine derivative of the present invention may be used alone or as a mixture with other materials for forming the hole injecting and transporting layer.
The material which can be used for forming the hole injecting and transporting layer as a mixture with the aromatic amine derivative of the present invention is not particularly limited as long as the material has a preferable property described above. The material can be arbitrarily selected from materials which are conventionally used as the charge transporting material of holes in photoconductive materials and known materials which are used for the hole injecting and transporting layer in organic EL devices.

Specific examples include: a triazole derivative (see, for example, US 3,112,197); an oxadiazole derivative (see, for example, US 3,189,447); an imidazole derivative (see, for example, JP-B 37-16096); a polyarylalkane derivative (see, for example, US 3,615,402, US 3,820,989, US 3,542,544, JP-B 45-555, JP-B 51-10983, JP-A 51-93224, JP-A 55-17105, JP-A 56-4148, JP-A 55-108667, JP-A 55-156953, and JP-A 56-36656); a pyrazoline derivative and a pyrazolone derivative (see, for example, US 3,180,729, US 4,278,746, JP-A 55-88064, JP-A 55-88065, JP-A 49-105537, JP-A 55-51086, JP-A 56-80051, JP-A 56-88141, JP-A 57-45545, JP-A 54-112637, and JP-A 55-74546); a phenylenediamine derivative (see, for example, US 3, 615, 404, JP-B 51-10105, JP-B 46-3712, JP-B 47-25336, JP-A 54-53435, JP-A 54-110536, and JP-A 54-119925); an arylamine derivative (see, for example, US 3, 567, 450, US 3,180,703, US 3, 240, 597, US 3, 658, 520, US 4,232,103, US 4,175,961, US 4,012,376, JP-B 49-35702, JP-B 39-27577, JP-A 55-144250, JP-A 56-119132, JP-A 56-22437, and DE 1,110,518); an amino-substituted chalcone derivative (see, for example, US 3,526,501); an oxazole derivative (those disclosed in US 3,257,203); a styrylanthracene derivative (see, for example, JP-A 56-46234); a fluorenone derivative (see, for example, JP-A 54-110837) ; a hydrazone derivative (see, for example, US 3,717,462, JP-A 54-59143, JP-A 55-52063, JP-A 55-52064, JP-A 55-46760, JP-A 55-85495, JP-A 57-11350, JP-A 57-148749, and JP-A 2-311591); a stilbene derivative (see, for example, JP-A 61-210363, JP-A 61-228451, JP-A 61-14642, JP-A 61-72255, JP-A 62-47646, JP-A 62-36674, JP-A 62-10652, JP-A 62-30255, JP-A 60-93445, JP-A 60-94462, JP-A 60-174749, and JP-A 60-175052); a silazane derivative (US 4,950,950); a polysilane-based copolymer (JP-A 2-204996); an aniline-based copolymer (JP-A 2-282263); and a conductive high molecular weight oligomer (particularly a thiophene oligomer) disclosed in JP-A 1-211399.

In addition to the above-mentioned materials which can be used as the material for the hole injecting and transporting layer, a porphyrin compound (those disclosed in, for example, JP-A 63-295695); an aromatic tertiary amine compound and a styrylamine compound (see, for example, US 4,127,412, JP-A 53-27033, JP-A 54-58445, JP-A 54-149634, JP-A 54-64299, JP-A 55-79450, JP-A 55-144250, JP-A 56-119132, JP-A 61-295558, JP-A 61-98353, and JP-A 63-295695) are preferable, and aromatic tertiary amines are particularly preferable.
Further examples of aromatic tertiary amine compounds include compounds having two fused aromatic rings in the molecule such as 4,4'-bis(N-(1-naphthyl)-N-phenylamino)-biphenyl (hereinafter referred to as NPD) as disclosed in US 5,061,569, and a compound in which three triphenylamine units are bonded together in a star-burst shape, such as 4,4',4"-tris(N-(3-methylphenyl)-N-phenylamino)-triphenylamine (hereinafter referred to as MTDATA) as disclosed in JP-A 4-308688.
Further, in addition to the aromatic dimethylidene-based compounds described above as the material for the light emitting layer, inorganic compounds such as Si of the p-type and SiC of the p-type can also be used as the material for the hole injecting and transporting layer.

The hole injecting and transporting layer can be formed by forming a thin layer from the aromatic amine derivative of the present invention in accordance with a known process such as the vacuum vapor deposition process, the spin coating process, the casting process and the LB process. The thickness of the hole injecting and transporting layer is not particularly limited. In general, the thickness is 5 nm to 5 µm. The hole injecting and transporting layer may be constituted of a single layer containing one or more materials described above or may be a laminate constituted of hole injecting and transporting layers containing materials different from the materials of the hole injecting and transporting layer described above as long as the aromatic amine derivative of the present invention is incorporated in the hole injecting and transporting zone.
Further, an organic semiconductor layer may be disposed as a layer for helping the injection of holes or electrons into the light emitting layer. As the organic semiconductor layer, a layer having a conductivity of 10⁻¹⁰ S/cm or greater is preferable. As the material for the organic semiconductor layer, oligomers containing thiophene, and conductive oligomers such as oligomers containing arylamine and conductive dendrimers such as dendrimers containing arylamine which are disclosed in JP-A 08-193191, can be used.

(6) Electron injecting and transporting layer Next, the electron injecting and transporting layer is a layer which helps injection of electrons into the light emitting layer, transports the holes to the light emitting region, and exhibits a great mobility of electrons. The adhesion improving layer is an electron injecting layer comprising a material exhibiting particularly improved adhesion with the cathode.
In addition, it is known that, in an organic EL device, emitted light is reflected by an electrode (cathode in this case), so emitted light directly extracted from an anode and emitted light extracted via the reflection by the electrode interfere with each other. The thickness of an electron transporting layer is appropriately selected from the range of several nanometers to several micrometers in order that the interference effect may be effectively utilized. When the thickness is particularly large, an electron mobility is preferably at least 10⁻⁵ cm²/Vs or more upon application of an electric field of 10⁴ to 10⁶ V/cm in order to avoid an increase in voltage.
A metal complex of 8-hydroxyquinoline or of a derivative of 8-hydroxyquinoline, or an oxadiazole derivative is suitable as a material to be used in an electron injecting layer. Specific examples of the metal complex of 8-hydroxyquinoline or of a derivative of 8-hydroxyquinoline that can be used as an electron injecting material include metal chelate oxynoid compounds each containing a chelate of oxine (generally 8-quinolinol or 8-hydroxyquinoline) such as tris(8-quinolinol)aluminum.

On the other hand, examples of the oxadiazole derivative include electron transfer compounds represented by the following general formulae: 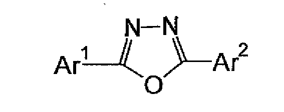 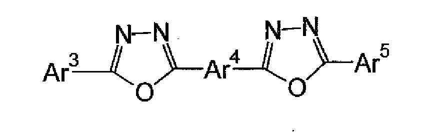 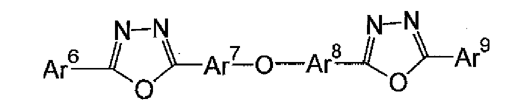 where: Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.
Examples of the aryl group include a phenyl group, a biphenyl group, an anthranyl group, a perylenyl group, and a pyrenyl group. Examples of the arylene group include a phenylene group, a naphthylene group, a biphenylene group, an anthranylene group, a perylenylene group, and a pyrenylene group. Examples of the substituent include alkyl groups having 1 to 10 carbon atoms, alkoxyl groups having 1 to 10 carbon atoms, and a cyano group. As the electron transfer compound, compounds which can form thin films are preferable.

Examples of the electron transfer compounds described above include the following. 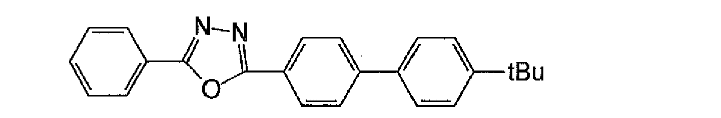 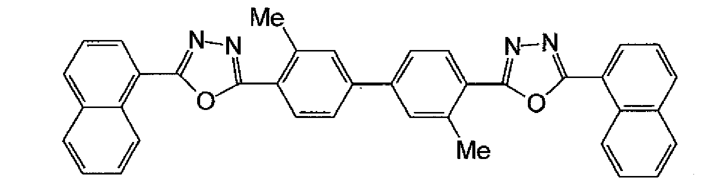 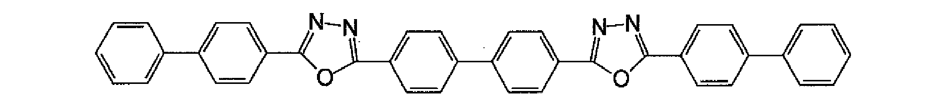 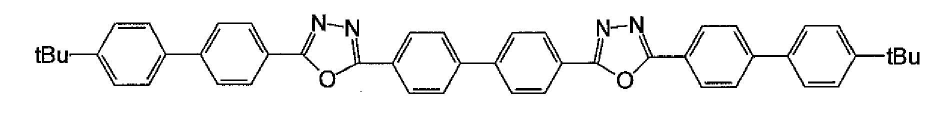 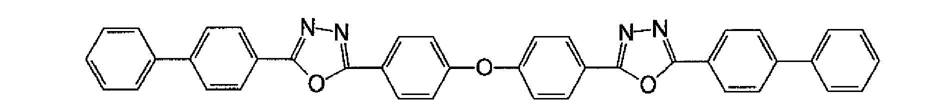 Further, materials represented by the following general formulae (A) to (F) can be used in an electron injecting layer and an electron transporting layer.

Nitrogen-containing heterocyclic derivatives represented by the general formulae (A) and (B): 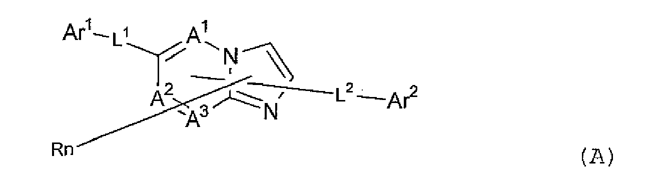 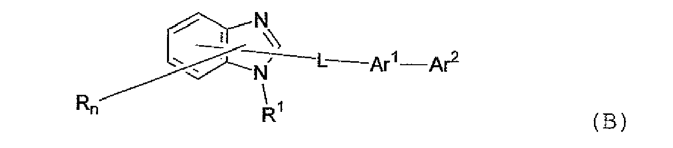 where:
A¹ to A³ each independently represent a nitrogen atom or a carbon atom;
Ar¹ represents a substituted or unsubstituted aryl group having 6 to 60 ring carbon atoms, or a substituted or unsubstituted heteroaryl group having 3 to 60 ring carbon atoms, Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms, or a divalent group of any one of themprovided that one of Ar¹ and Ar² represents a substituted or unsubstituted fused ring group having 10 to 60 ring carbon atoms or a substituted or unsubstituted monohetero fused ring group having 3 to 60 ring carbon atoms, or a divalent group of one of them;
L¹, L², and L each independently represent a single bond, a substituted or unsubstituted arylene group having 6 to 60 ring carbon atoms, a substituted or unsubstituted heteroarylene group having 3 to 60 ring carbon atoms, or a substituted or unsubstituted fluorenylene group;
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms. n represents an integer of 0 to 5, and, when n represents 2 or more, multiple R's may be identical to or different from each other, and multiple R groups adjacent to each other may be bonded to each other to form a carbocyclic aliphatic ring or a carbocyclic aromatic ring; and
R¹ represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxy group having 1 to 20 carbon atoms, or -L-Ar¹-Ar².

A nitrogen-containing heterocyclic ring derivative represented by the general formula (C):

HAr-L-Ar¹-Ar² (C)

where: HAr represents a nitrogen-containing heterocyclic ring having 3 to 40 carbon atoms which may have a substituent, L represents a single bond, an arylene group having 6 to 60 carbon atoms which may have a substituent, a heteroarylene group having 3 to 60 carbon atoms which may have a substituent, or a fluorenylene group which may have a substituent, Ar¹ represents a divalent aromatic hydrocarbon group having 6 to 60 carbon atoms which may have a substituent, and Ar² represents an aryl group having 6 to 60 carbon atoms which may have a substituent, or a heteroaryl group having 3 to 60 carbon atoms which may have a substituent.

A silacyclopentadiene derivative represented by the general formula (D):

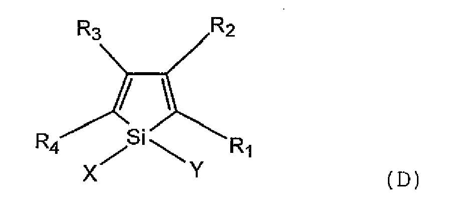

where: X and Y each independently represent a saturated or unsaturated hydrocarbon group having 1 to 6 carbon atoms, an alkoxy group, an alkenyloxy group, an alkynyloxy group, a hydroxy group, a substituted or unsubstituted aryl group, or a substituted or unsubstituted heterocycle, or X and Y are bonded to each other to form a structure as a saturated or unsaturated ring; and R₁ to R₄ each independently represent hydrogen, a halogen atom, a substituted or unsubstituted alkyl group having 1 to 6 carbon atoms, an alkoxy group, an aryloxy group, a perfluroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or, a structure in which a substituted or unsubstituted ring is condensed when two or more of R₁ to R₄ are adjacent to each other.

A borane derivative represented by the general formula (E) :

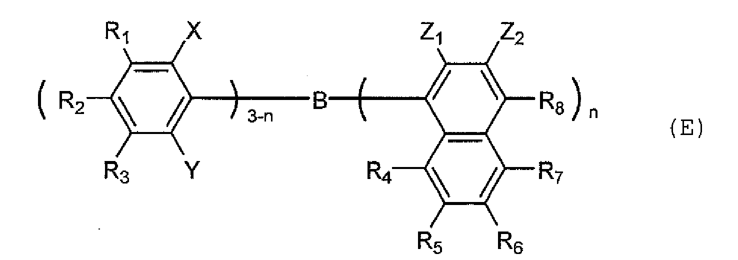

where: R₁ to R₈ and Z₂ each independently represent a hydrogen atom, a saturated or unsaturated hydrocarbon group, an aromatic group, a heterocyclic group, a substituted amino group, a substituted boryl group, an alkoxy group, or an aryloxy group; X, Y, and Z₁ each independently represent a saturated or unsaturated hydrocarbon group, an aromatic group, a heterocyclic group, a substituted amino group, an alkoxy group, or an aryloxy group; substituents of Z₁ and Z₂ may be bonded to each other to form a fused ring; and n represents an integer of 1 to 3, and, when n represents 2 or more, Z₁' s may be different from each other provided that the case where n represents 1, X, Y, and R₂ each represent a methyl group, R₈ represents a hydrogen atom or a substituted boryl group and the case where n represents 3 and Z₁'s each represent a methyl group are excluded.

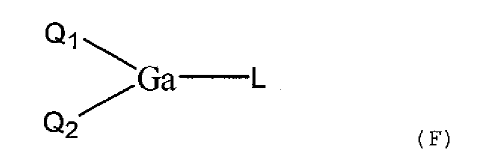

where: Q¹ and Q² each independently represent a ligand represented by the following general formula (G) ; and L represents a halogen atom, a substituted or unsubstituted alkyl group, a substituted or unsubstituted cycloalkyl group, a substituted or unsubstituted aryl group, a substituted or unsubstituted heterocyclic group, -OR¹ where R¹ represents a hydrogen atom, a substituted or unsubstituted alkyl group, a substituted or unsubstituted cycloalkyl group, a substituted or unsubstituted aryl group, or a substituted or unsubstituted heterocyclic group, or a ligand represented by -O-Ga-Q³(Q⁴) where Q³ and Q⁴ are identical to Q¹ and Q², respectively.

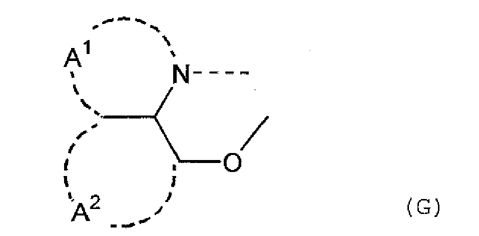

where rings A¹ and A² are six-membered aryl ring structures which are condensed with each other and each of which may have a substituent.

The metal complex behaves strongly as an n-type semiconductor, and has a large electron injecting ability. Further, generation energy upon formation of the complex is low. As a result, the metal and the ligand of the formed metal complex are bonded to each other so strongly that the fluorescent quantum efficiency of the complex as a light emitting material improves.
Specific examples of a substituent in the rings A¹ and A² which each form a ligand in the general formula (G) include: a halogen atom such as chlorine, bromine, iodine, or fluorine; a substituted or unsubstituted alkyl group such as a methyl group, an ethyl group, a propyl group, a butyl group, an s-butyl group, a t-butyl group, a pentyl group, a hexyl group, a heptyl group, an octyl group, a stearyl group, or trichloromethyl group; a substituted or unsubstituted aryl group such as a phenyl group, a naphtyl group, a 3-methylphenyl group, a 3-methoxyphenyl group, a 3-fluorophenyl group, a 3-trichloromethylphenyl group, a 3-trifluoromethylphenyl group, or a 3-nitrophenyl group; a substituted or unsubstituted alkoxy group such as a methoxy group, an n-butoxy group, a t-butoxy group, a trichloromethoxy group, a trifluoroethoxy group, a pentafluoropropoxy group, a 2,2,3,3-tetrafluoropropoxy group, an 1,1,1,3,3,3-hexafluoro-2-propoxy group, or a 6-(perfluoroethyl)hexyloxy group; a substituted or unsubstituted aryloxy group such as a phenoxy group, a p-nitrophenoxy group, p-t-butylphenoxy group, a 3-fluorophenoxy group, a pentafluorophenyl group, or a 3-trifluoromethylphenoxy group; a substituted or unsubstituted alkylthio group such as a methylthio group, an ethylthio group, a t-butylthio group, a hexylthio group, an octylthio group, or a trifluoromethylthio group; a substituted or unsubstituted arylthio group such as a phenylthio group, a p-nitrophenylthio group, a p-t-butylphenylthio group, a 3-fluorophenylthio group, a pentafluorophenylthio group, or a 3-trifluoromethylphenylthio group; a mono-substituted or di-substituted amino group such as a cyano group, a nitro group, an amino group, a methylamino group, a dimethylamino group, an ethylamino group, a diethylamino group, a dipropylamino group, a dibutylamino group, or a diphenylamino group; an acylamino group such as a bis (acetoxymethyl) amino group, a bis (acetoxyethyl) amino group, a bis (acetoxypropyl) amino group, or a bis (acetoxybutyl) amino group; a carbamoyl group such as a hydroxyl group, a siloxy group, an acyl group, a methylcarbamoyl group, a dimethylcarbamoyl group, an ethylcarbamoyl group, a diethylcarbamoyl group, a propylcarbamoyl group, a butylcarbamoyl group, or a phenylcarbamoyl group; a cycloalkyl group such as a carboxylic acid group, a sulfonic acid group, an imide group, a cyclopentane group, or a cyclohexyl group; an aryl group such as a phenyl group, a naphthyl group, a biphenyl group, an anthranyl group, a phenanthryl group, a fluorenyl group, or a pyrenyl group; and a heterocyclic group such as a pyridinyl group, a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triathinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzoimidazolyl group, or a puranyl group. In addition, the above-mentioned substituents may be bound to each other to further form a six-membered aryl ring or a heterocycle.

A preferable embodiment of the organic EL device of the present invention includes an element including a reducing dopant in the region of electron transport or in the interfacial region of the cathode and the organic thin film layer. The reducing dopant is defined as a substance which can reduce a compound having the electron-transporting property. Various compounds can be used as the reducing dopant as long as the compounds have a uniform reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.
More specifically, examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV), and Cs (the work function: 1.95 eV) and at least one alkaline earth metal selected from the group consisting of Ca (the work function: 2.9 eV), Sr (the work function: 2.0 to 2.5 eV), and Ba (the work function: 2.52 eV) . Among the above-mentioned substances, at least one alkali metal selected from the group consisting of K, Rb, and Cs is more preferable, Rb and Cs are still more preferable, and Cs is most preferable as the reducing dopant. Those alkali metals have great reducing ability, and the luminance of the emitted light and the life time of the organic EL device can be increased by addition of a relatively small amount of the alkali metal into the electron injecting zone. As the reducing dopant having a work function of 2.9 eV or smaller, combinations of two or more alkali metals thereof are also preferable. Combinations having Cs such as the combinations of Cs and Na, Cs and K, Cs and Rb, and Cs, Na, and K are more preferable. The reducing ability can be efficiently exhibited by the combination having Cs. The luminance of emitted light and the life time of the organic EL device can be increased by adding the combination having Cs into the electron injecting zone.

The present invention may further include an electron injecting layer which is composed of an insulating material or a semiconductor and disposed between the cathode and the organic layer. At this time, leak of electric current can be effectively prevented by the electron injecting layer and the electron injecting property can be improved. As the insulating material, at least one metal compound selected from the group consisting of alkali metal chalcogenides, alkaline earth metal chalcogenides, alkali metal halides, and alkaline earth metal halides is preferable. It is preferable that the electron injecting layer be composed of the above-mentioned substance such as the alkali metal chalcogenide since the electron injecting property can be further improved. Preferable examples of the alkali metal chalcogenide include Li₂O, K₂O, Na₂S, Na₂Se, and Na₂O. To be specific, preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such as CaF₂, BaF₂, SrF₂, MgF₂, and BeF₂ and halides other than the fluorides.
Examples of the semiconductor composing the electron-transporting layer include oxides, nitrides, and oxide nitrides of at least one element selected from Ba, Ca, Sr, Yb, Al, Ga, In, Li, Na, Cd, Mg, Si, Ta, Sb, and Zn used alone or in combination of two or more. It is preferable that the inorganic compound composing the electron-transporting layer form a crystallite or amorphous insulating thin film. When the electron injecting layer is composed of the insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

### (7) Cathode

As the cathode, amaterial such as ametal, analloy, a conductive compound, or a mixture of those materials which has a small work function (4 eV or smaller) is used because the cathode is used for injecting electrons to the electron injecting and transporting layer or the light emitting layer. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of the electrode material described above in accordance with a process such as the vapor deposition process and the sputtering process.
When the light emitted from the light emitting layer is obtained through the cathode, it is preferable that the cathode have a transmittance of the emitted light greater than 10%.
It is also preferable that the sheet resistivity of the cathode is several hundred Ω/□ or smaller. The thickness of the cathode is, in general, selected in the range of 10 nm to 1 µm and preferably in the range of 50 to 200 nm.

### (8) Insulating layer

Defects in pixels tend to be formed in organic EL device due to leak and short circuit since an electric field is applied to ultra-thin films. To prevent the formation of the defects, a layer of a thin film having an insulating property may be inserted between the pair of electrodes.
Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned compounds may also be used.

### (9) Method of producing the organic EL device

To prepare the organic EL device of the present invention, the anode and the light emitting layer, and, where necessary, the hole injecting and the transporting layer and the electron injecting and transporting layer are formed in accordance with the illustrated process using the illustrated materials, and the cathode is formed in the last step. The organic EL device may also be prepared by forming the above-mentioned layers in the order reverse to that described above, i.e., the cathode being formed in the first step and the anode in the last step.
Hereinafter, an embodiment of the process for preparing an organic EL device having a construction in which an anode, a hole injecting layer, a light emitting layer, an electron injecting layer, and a cathode are disposed successively on a substrate transmitting light will be described.
On a suitable transparent substrate, a thin film made of a material for the anode is formed in accordance with the vapor deposition process or the sputtering process so that the thickness of the formed thin film is 1µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process, or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions be suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the compound to be used (i.e., the material for the hole injecting layer) and the crystal structure and the recombination structure of the target hole injecting layer.

Then, the light emitting layer is formed on the hole injecting layer formed above. A thin film of the organic light emitting material can be formed by using a desired organic light emitting material in accordance with a process such as the vacuum vapor deposition process, the sputtering process, the spin coating process, or the casting process. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer, although the conditions are different depending on the used compound.
Next, an electron injecting layer is formed on the light emitting layer formed above. Similarly to the hole injecting layer and the light emitting layer, it is preferable that the electron injecting layer be formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.
When the vapor deposition process is used, the aromatic amine derivative of the present invention can be deposited by vapor in combination with other materials, although the situation may be different depending on which layer in the light emitting zone or in the hole transporting zone comprises the compound. When the spin coating process is used, the compound can be incorporated into the formed layer by using a mixture of the compound with other materials.
A cathode is formed on the electron injecting layer formed above in the last step, and an organic EL device can be obtained.
The cathode is made of a metal and can be formed in accordance with the vacuum vapor deposition process or the sputtering process. It is preferable that the vacuum vapor deposition process be used in order to prevent formation of damages on the lower organic layers during the formation of the film.
In the above-mentioned preparation of the organic EL device, it is preferable that the above-mentioned layers from the anode to the cathode be formed successively while the preparation system is kept in a vacuum after being evacuated once.

The method of forming the layers in the organic EL device of the present invention is not particularly limited. A conventionally known process such as the vacuum vapor deposition process and the spin coating process can be used. The organic thin film layer which is used in the organic EL device of the present invention and comprises the compound represented by general formula (1) described above can be formed in accordance with a known process such as the vacuum vapor deposition process and the molecular beam epitaxy process (the MBE process) or, using a solution prepared by dissolving the compounds into a solvent, in accordance with a coating process such as the dipping process, the spin coatingprocess, the casting process, the bar coating process, or the roll coating process.
The thickness of each layer in the organic thin film layer in the organic EL device of the present invention is not particularly limited. In general, an excessively thin layer tends to have defects such as pin holes, and an excessively thick layer requires a high applied voltage to decrease the efficiency. Therefore, a thickness in the range of several nanometers to 1 µm is preferable.
The organic EL device which can be prepared as described above emits light when a direct voltage of 5 to 40 V is applied in the condition that the anode is connected to a positive electrode (+) and the cathode is connected to a negative electrode (-) . When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### [EXAMPLES]

Hereinafter, the present invention will be described in more detail on the basis of synthesis examples and examples.

### Synthesis Example 1 (synthesis of Intermediate 1)

In a stream of argon, 5 . 7 g of benzamide (manufactured by Tokyo Chemical Industry Co., Ltd.), 10 g of 4-bromobiphenyl (manufactured by Tokyo Chemical Industry Co., Ltd.), 0.82 g of cuprous iodide (manufactured by HIROSHIMA WAKO CO., LTD.), 0.76 g of N,N'-dimethylethylenediamine (manufactured by Aldrich), 11. 8 g of potassium carbonate (manufactured by HIROSHIMA WAKO CO., LTD.), and 60 ml of xylene were loaded into a 200-ml three-necked flask, and the whole was reacted at 130°C for 36 hours.
After having been cooled, the resultant was filtered and washed with toluene. Further, the resultant was washed with water and methanol, and was then dried, thereby resulting in Intermediate 1 to be described below as 10.5 g of pale yellow powder. The powder was identified as Intermediate 1 to be described below because a main peak of m/z = 273 was obtained for C₁₉H₁₅NO = 273 as a result of field desorption mass spectrum (FD-MS) analysis.

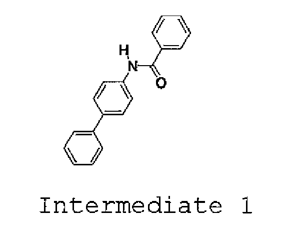

### Synthesis Example 2 (synthesis of Intermediate 2)

In a stream of argon, 20.7 g of 1-bromonaphthalene, 80 ml of dehydrated ether, and 80 ml of dehydrated toluene were loaded into a 500-ml three-necked flask. 120 mmol of a solution of n-butyllithium (n-BuLi) in hexane were charged into the resultant at -30°C, and the whole was reacted at 0°C for 1 hour. The temperature of the resultant was cooled to -70°C, 70 ml of triisopropyl borate (B(OiPr)₃) were loaded into the resultant, the temperature of the mixture was slowly increased to room temperature, and the mixture was stirred for 1 hour. 80 ml of 10% hydrochloric acidwere added to the resultant, and the whole was extracted with ethyl acetate/water and dried with anhydrous sodium sulfate. The solution was concentrated and washed with hexane, whereby 11.7 g of a boronic acid compound were obtained.
In a streamof argon, 19.3 g of the boronic acid compound obtained in the foregoing, 26.5 g of 4-iodobromobenzene, 3.8 g of tetrakis (triphenylphosphine)palladium (Pd(PPh₃)₄), 100 ml of a 2-M sodium carbonate (Na₂CO₃) solution, and 160 ml of dimethoxyethane were loaded into a 500-ml three-necked flask, and then the whole was refluxed for 8 hours. The reaction liquid was extracted with toluene/water and dried with anhydrous sodium sulfate. The resultant was concentrated under reduced pressure, and the resultant coarse product was subjected to column purification, thereby resulting in Intermediate 2 to be described below as 17.6 g of white powder. The powder was identified as Intermediate 2 to be described below because main peaks of m/z = 282 and 284 were obtained for C₁₆H₁₁Br = 283 as a result of FD-MS analysis.

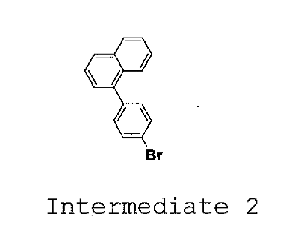

### Synthesis Example 3 (synthesis of Intermediate 3)

A reaction was performed in the same manner as in Synthesis Example 2 except that 20.7 g of 2-bromonaphthalene were used instead of 20.7 g of 1-bromonaphthalene, thereby resulting in Intermediate 3 to be described below as 17.9 g of white powder. The powder was identified as Intermediate 3 to be described below because main peaks of m/z = 282 and 284 were obtained for C₁₆H₁₁Br = 283 as a result of FD-MS analysis.

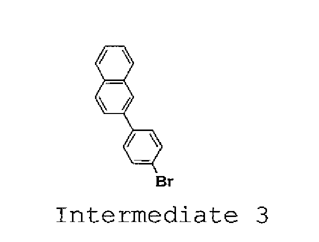

### Synthesis Example 4 (synthesis of Intermediate 4)

A reaction was performed in the same manner as in Synthesis Example 2 except that 25.7 g of 9-bromophenanthrene were used instead of 20.7 g of 1-bromonaphthalene, thereby resulting in Intermediate 3 to be described below as 20.5 g of white powder. The powder was identified as Intermediate 4 to be described below because main peaks of m/z = 332 and 334 were obtained for C₂₀H₁₃Br = 333 as a result of FD-MS analysis.

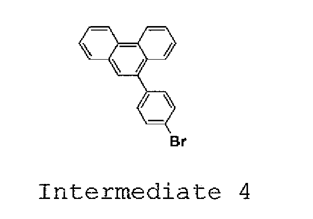

### Synthesis Example 5 (synthesis of Intermediate 5)

In a stream of argon, 16.9 g of Intermediate 1, 21.1 g of Intermediate 2, 1.14 g of cuprous iodide (manufactured by HIROSHIMA WAKO CO., LTD.), 1.06 g of N,N'-dimethylethylenediamine (manufactured by Aldrich), 20.0 g of potassium carbonate (manufactured by HIROSHIMA WAKO CO., LTD.), and 100 ml of xylene were loaded into a 300-ml three-necked flask, and the whole was reacted at 130°C for 36 hours.
After having been cooled, the resultant was filtered and washed with toluene. Further, the resultant was washed with water and methanol, and was then dried, whereby 23.5 g of pale yellow powder were obtained.
18.0 g of the above-mentioned powder, 15.1 g of potassium hydroxide (manufactured by HIROSHIMA WAKO CO., LTD.), 13 ml of ion-exchanged water, 17 ml of xylene (manufactured by HIROSHIMA WAKO CO., LTD.), and 9 ml of ethanol (manufactured by HIROSHIMA WAKO CO., LTD.) were loaded into a 300-ml three-necked flask, and then the whole was refluxed for 36 hours. After the completion of the reaction, the resultant was extracted with toluene and dried with magnesium sulfate. The resultant was concentrated under reduced pressure, and the resultant coarse product was subjected to column purification. Then, the resultant was recrystallized with toluene, and the recrystallized product was separated by filtration and dried, thereby resulting in Intermediate 5 to be described below as 13.8 g of white powder. The powder was identified as Intermediate 5 to be described below because a main peak of m/z = 371 was obtained for C₂₈H₂₁N = 371 as a result of FD-MS analysis.

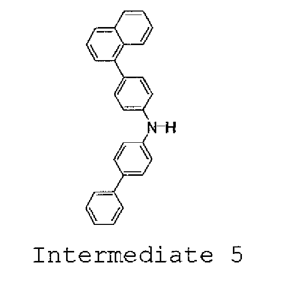

### Synthesis Example 6 (synthesis of Intermediate 6)

A reaction was performed in the same manner as in Synthesis Example 5 except that 21.1 g of Intermediate 3 were used instead of 21.1 g of Intermediate 2, thereby resulting in Intermediate 6 to be described below as 14.6 g of white powder. The powder was identified as Intermediate 6 to be described below because a main peak of m/z = 371 was obtained for C₂₈H₂₁N = 371 as a result of FD-MS analysis.

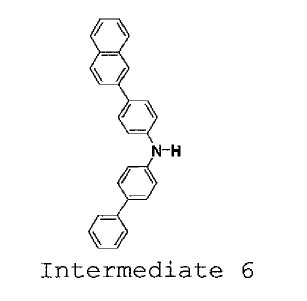

### Synthesis Example 7 (synthesis of Intermediate 7)

A reaction was performed in the same manner as in Synthesis Example 5 except that 4.2 g of benzamide were used instead of 16. 9 g of Intermediate 1, thereby resulting in Intermediate 7 to be described below as 8.5 g of white powder. The powder was identified as Intermediate 7 to be described below because a main peak of m/z = 421 was obtained for C₃₂H₂₃N = 421 as a result of FD-MS analysis.

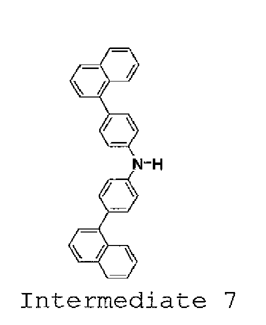

### Synthesis Example 8 (synthesis of Intermediate 8.)

A reaction was performed in the same manner as in Synthesis Example 5 except that 11.5 g of 1-acetamidonaphthalene were used instead of 16.9 g of Intermediate 1, thereby resulting in Intermediate 8 to be described below as 12.8 g of white powder. The powder was identified as Intermediate 8 to be described below because a main peak of m/z = 345 was obtained for C₂₆H₁₉N = 345 as a result of FD-MS analysis.

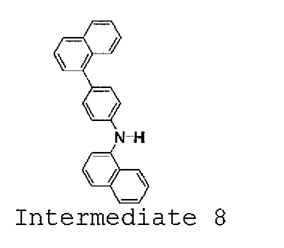

### Synthesis Example 9 (synthesis of Intermediate 9)

In a stream of argon, 5.5 g of aniline, 14.2 g of Intermediate 2, 6.8 g of t-butoxysodium (manufactured by HIROSHIMA WAKO CO., LTD.), 0.46 g of tris(dibenzylideneacetone)dipalladium(0) (manufactured by Aldrich), and 300 ml of dehydrated toluene were loaded, and the whole was reacted at 80°C for 8 hours.
After having been cooled, 500 ml of water were added to the resultant, and the mixture was subjected to Celite filtration. The filtrate was extracted with toluene and dried with anhydrous magnesium sulfate. The resultant was concentrated under reduced pressure, and the resultant coarse product was subjected to column purification. Then, the resultant was recrystallized with toluene, and the recrystalli zedproduct was separated by filtration and dried, thereby resulting in 11.8 g of pale yellow powder. The powder was identified as Intermediate 9 to be described below because a main peak of m/z = 295 was obtained for C₂₂H₁₇N = 295 as a result of FD-MS analysis.

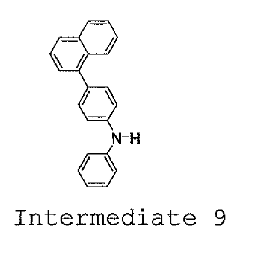

### Synthesis Example 10 (synthesis of Intermediate 10)

A reaction was performed in the same manner as in Synthesis Example 9 except that 14.2 g of Intermediate 3 were used instead of 14.2 g of Intermediate 2, thereby resulting in Intermediate 10 to be described below as 12.3 g of pale yellow powder. The powder was identified as Intermediate 10 to be described below because a main peak of m/z = 295 was obtained for C₂₂H₁₇N = 295 as a result of FD-MS analysis.

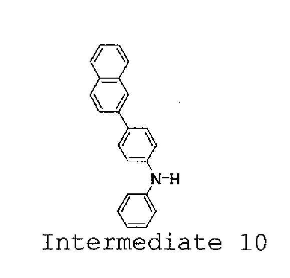

### Synthesis Example 11 (synthesis of Intermediate 11)

A reaction was performed in the same manner as in Synthesis Example 9 except that 16.7 g of Intermediate 4 were used instead of 14.2 g of Intermediate 2, thereby resulting in Intermediate 11 to be described below as 13.3 g of pale yellow powder. The powder was identified as Intermediate 11 to be described below because a main peak of m/z = 345 was obtained for C₂₆H₁₉N = 345 as a result of FD-MS analysis.

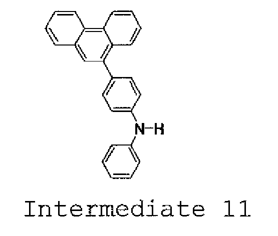

### Synthesis Example 12 (synthesis of Intermediate 12)

In a stream of argon, 4. 7 g of 4-bromobiphenyl, 23 g of iodine, 9.4 g of periodic acid dihydrate, 42 ml of water, 360 ml of acetic acid, and 11 ml of sulfuric acid were loaded into a 1,000-ml three-necked flask, and the whole was stirred at 65°C for 30 minutes. After that, the resultant was reacted at 90°C for 6 hours. The reactant was injected into ice water, followed by filtration. The resultant was washed with water, and was then washed with methanol, thereby resulting in Intermediate 12 to be described below as 18 g of white powder. The powder was identified as Intermediate 12 to be described below because main peaks of m/z = 358 and 360 were obtained for C₁₂H₈BrI = 359 as a result of FD-MS analysis.

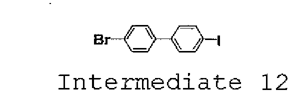

### Synthesis Example 13 (synthesis of Intermediate 13)

In a stream of argon, 20.7 g of 1-bromonaphthalene, 80 ml of dehydrated ether, and 80 ml of dehydrated toluene were loaded into a 500-ml three-necked flask. 120 mmol of a solution of n-BuLi in hexane were charged into the resultant at -30°C, and the whole was reacted at 0°C for 1 hour. The temperature of the resultant was cooled to -70°C, 70 ml of triisopropyl borate (B(OiPr)₃) were loaded into the resultant, the temperature of the mixture was slowly increased to room temperature, and the mixture was stirred for 1 hour. 80 ml of 10% hydrochloric acid were added to the resultant, and the whole was extracted with ethyl acetate/water and dried with anhydrous sodium sulfate. The solution was concentrated and washed with hexane, whereby 11.7 g of a boronic acid compound were obtained.
In a streamof argon, 17.2 g of the boronic acid compound obtained in the foregoing, 39.5 g of Intermediate 12, 3.8 g of tetrakis (triphenylphosphine)palladium (Pd(PPh₃)₄), 100 ml of a 2-M sodium carbonate (Na₂CO₃) solution, and 160 ml of dimethoxyethane were loaded into a 500-ml three-necked flask, and then the whole was refluxed for 8 hours. The reaction liquid was extracted with toluene/water and dried with anhydrous sodium sulfate. The resultant was concentrated under reduced pressure, and the resultant coarse product was subjected to column purification, thereby resulting in Intermediate 13 to be described below as 21.5 g of white powder. The powder was identified as Intermediate 13 to be described below because main peaks of m/z = 358 and 360 were obtained for C₂₂H₁₅Br = 359 as a result of FD-MS analysis.

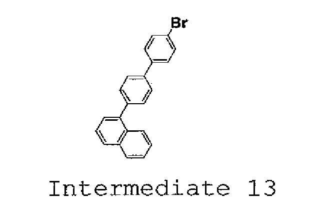

### Synthesis Example 14 (synthesis of Intermediate 14)

A reaction was performed in the same manner as in Synthesis Example 13 except that 20.7 g of 2-bromonaphthalene were used instead of 20.7 g of 1-bromonaphthalene, thereby resulting in Intermediate 14 to be described below as 17.1 g of white powder. The powder was identified as Intermediate 14 to be described below because main peaks of m/z = 358 and 360 were obtained for C₂₂H₁₅Br = 359 as a result of FD-MS analysis.

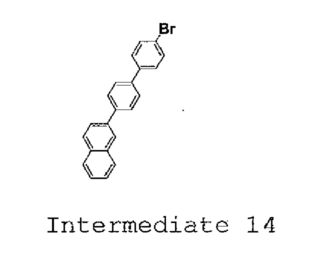

### Synthesis Example 15 (synthesis of Intermediate 15)

A reaction was performed in the same manner as in Synthesis Example 9 except that 17.9 g of Intermediate 13 were used instead of Intermediate 2, thereby resulting in 11. 2 g of pale yellow powder. The powder was identified as Intermediate 15 to be described below because a main peak of m/z = 371 was obtained for C₂₈H₂₁N = 371 as a result of FD-MS analysis.

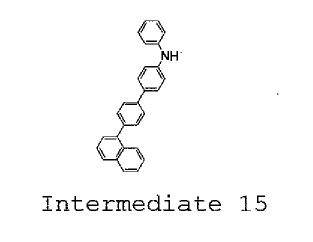

### Synthesis Example 16 (synthesis of Intermediate 16)

A reaction was performed in the same manner as in Synthesis Example 9 except that 17.9 g of Intermediate 14 were used instead of Intermediate 2, thereby resulting in 12. 5 g of pale yellow powder. The powder was identified as Intermediate 16 to be described below because a main peak of m/z = 371 was obtained for C₂₈H₂₁N = 371 as a result of FD-MS analysis.

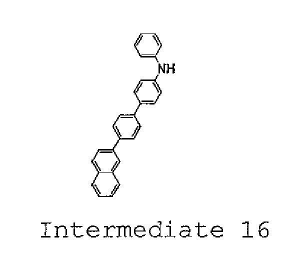

### Example-of-synthesis 1 (synthesis of Compound H1)

In a stream of argon, 3.2 g of 4-4'-diiodobiphenyl, 6.5 g of Intermediate 5, 2.1 g of t-butoxysodium (manufactured by HIROSHIMA WAKO CO., LTD.), 71 mg of tris(dibenzylideneacetone)dipalladium(0) (manufactured by Aldrich), 40 mg of tri-t-butylphosphine, and 100 ml of dehydrated toluene were loaded, and the whole was reacted at 80°C for 8 hours.
After having been cooled, 500 ml of water were added to the resultant, and the mixture was subjected to Celite filtration. The filtrate was extracted with toluene and dried with anhydrous magnesium sulfate. The resultant was concentrated under reduced pressure, and the resultant coarse product was subjected to column purification. Then, the resultant was recrystallized with toluene, and the recrystallizedproduct was separated by filtration and dried, thereby resulting in 4.8 g of pale yellow powder. The powder was identified as Compound H1 to be described below because a main peak of m/z = 892 was obtained for C₆₈H₄₈N₂ = 892 as a result of field desorption mass spectrometry (FD-MS) analysis.

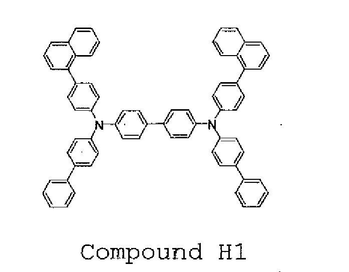

### Example-of-synthesis 2 (synthesis of Compound H2)

A reaction was performed in the same manner as in Example-of-synthesis 1 except that 6.5 g of Intermediate 6 were used instead of Intermediate 5, thereby resulting in 4.2 g of pale yellow powder. The powder was identified as Compound H2 to be described below because a main peak of m/z = 892 was obtained for C₆₈H₄₈N₂ = 892 as a result of FD-MS analysis.

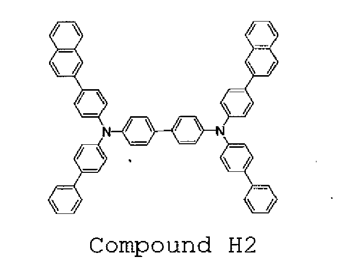

### Example-of-synthesis 3 (synthesis of Compound H3)

A reaction was performed in the same manner as in Example-of-synthesis 1 except that 7.4 g of Intermediate 7 were used instead of Intermediate 5, thereby resulting in 5.4 g of pale yellow powder. The powder was identified as Compound H3 to be described below because a main peak of m/z = 992 was obtained for C₇₆H₅₂N₂ = 992 as a result of FD-MS analysis.

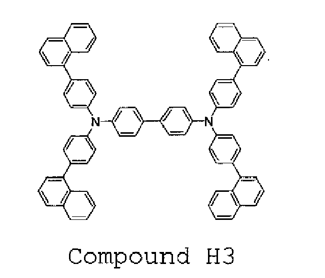

### Example-of-synthesis 4 (synthesis of Compound H4)

A reaction was performed in the same manner as in Example-of-synthesis 1 except that 6.0 g of Intermediate 8 were used instead of Intermediate 5, thereby resulting in 5.2 g of pale yellow powder. The powder was identified as Compound H4 to be described below because a main peak of m/z = 840 was obtained for C₆₄H₄₄N₂ = 840 as a result of FD-MS analysis.

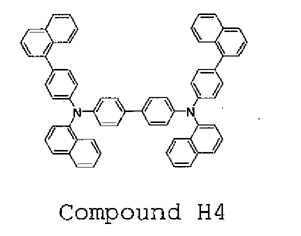

### Example-of-synthesis 5 (synthesis of Compound H5)

A reaction was performed in the same manner as in Example-of-synthesis 1 except that 5.2 g of Intermediate 9 were used instead of Intermediate 5, thereby resulting in 3.7 g of pale yellow powder. The powder was identified as Compound H5 to be described below because a main peak of m/z = 740 was obtained for C₅₆H₄₀N₂ = 740 as a result of FD-MS analysis.

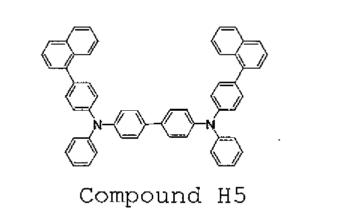

### Example-of-synthesis 6 (synthesis of Compound H6)

A reaction was performed in the same manner as in Example-of-synthesis 1 except that 5.2 g of Intermediate 10 were used instead of Intermediate 5, thereby resulting in 3.9 g of pale yellow powder. The powder was identified as Compound H6 to be described below because a main peak of m/z = 740 was obtained for C₅₆H₄₀N₂ = 740 as a result of FD-MS analysis.

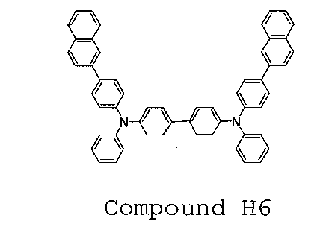

### Example-of-synthesis 7 (synthesis of Compound H7)

A reaction was performed in the same manner as in Example-of-synthesis 1 except that 6.5 g of Intermediate 11 were used instead of Intermediate 5, thereby resulting in 4.4 g of pale yellow powder. The powder was identified as Compound H7 to be described below because a main peak of m/z = 840 was obtained for C₆₄H₄₄N₂ = 840 as a result of FD-MS analysis.

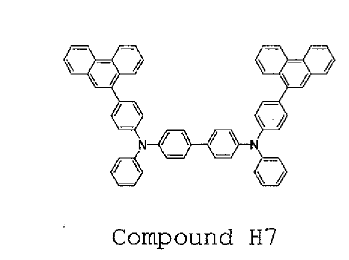

### Example-of-synthesis 8 (synthesis of Compound H8)

A reaction was performed in the same manner as in Example-of-synthesis 5 except that 3.1 g of 4-4'-dibromoterphenyl were used instead of 4-4'-diiodobiphenyl, thereby resulting in 4.1 g of pale yellow powder. The powder was identified as Compound H8 to be described below because a main peak of m/z = 816 was obtained for C₆₂H₄₄N₂ = 816 as a result of FD-MS analysis.

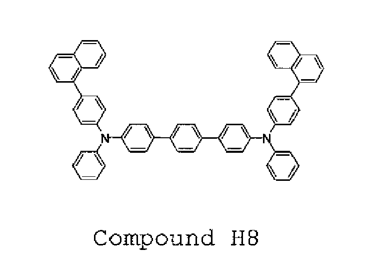

### Example-of-synthesis 9 (synthesis of Compound H9)

A reaction was performed in the same manner as in Example-of-synthesis 1 except that 3.1 g of 4-4'-dibromoterphenyl were used instead of 4-4'-diiodobiphenyl, thereby resulting in 5.2 g of pale yellow powder. The powder was identified as Compound H9 to be described below because a main peak of m/z = 968 was obtained for C₇₄H₅₂N₂ = 968 as a result of FD-MS analysis.

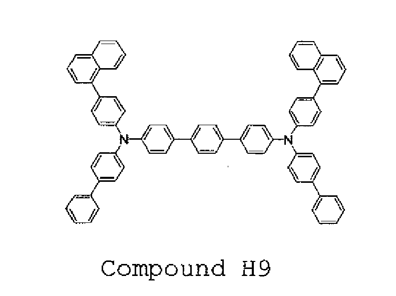

### Example-of-synthesis 10 (synthesis of Compound H10)

A reaction was performed in the same manner as in Example-of-synthesis 1 except that 6.5 g of Intermediate 15 were used instead of Intermediate 5, thereby resulting in 4.2 g of pale yellow powder. The powder was identified as Compound H10 to be described below because a main peak of m/z = 892 was obtained for C₆₈H₄₈N₂ = 892 as a result of FD-MS analysis.

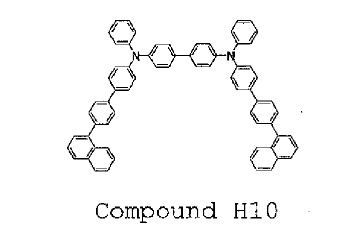

### Example-of-synthesis 11 (synthesis of Compound H11)

A reaction was performed in the same manner as in Example-of-synthesis 1 except that 6.5 g of Intermediate 16 were used instead of Intermediate 5, thereby resulting in 4.0 g of pale yellow powder. The powder was identified as Compound H11 to be described below because a main peak of m/z = 892 was obtained for C₆₈H₄₈N₂ = 892 as a result of FD-MS analysis.

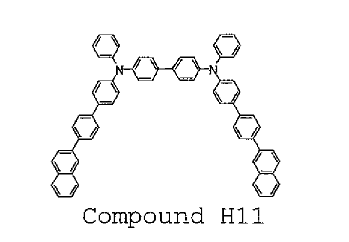

### Example 1 (production of organic EL device)

A glass substrate with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes.
The glass substrate with the transparent electrode line after the washing was mounted on a substrate holder of a vacuum deposition device. First, Compound H232 to be described below was formed into a film having a thickness of 60 nm on the surface on the side where the transparent electrode line was formed to cover the transparent electrode. The H232 film functions as a hole injecting layer. Compound H1 described above, as a hole transporting material, was formed into a film having a thickness of 20 nm on the H232 film. The film functions as a hole transporting layer. Further, Compound EM1 to be described below was deposited from the vapor and formed into a film having a thickness of 40 nm. Simultaneously with this formation, Amine Compound D1 having a styryl group to be described below, as a light emitting molecule, was deposited from the vapor in such a manner that a weight ratio between Compound EM1 and Amine Compound D1 would be 40 : 2. The film functions as a light emitting layer.
Alq to be described below was formed into a film having a thickness of 10 nm on the resultant film. The film functions as an electron injecting layer. After that, Li serving as a reducing dopant (Li source: manufactured by SAES Getters) and Alq were subjected to co-deposition. Thus, anAlq:Li film (having a thickness of 10 nm) was formed as an electron injecting layer (cathode). Metal A1 was deposited from the vapor onto the Alq:Li film to form a metal cathode. Thus, an organic EL device was formed.
In addition, the current efficiency of the resultant organic EL device was measured, and the luminescent color of the device was observed. A current efficiency at 10 mA/cm² was calculated by measuring a luminance by using a CS1000 manufactured by Minolta. Further, the half lifetime of light emission in DC constant current driving at an initial luminance of 5,000 cd/m² and room temperature was measured. Table 1 shows the results thereof.

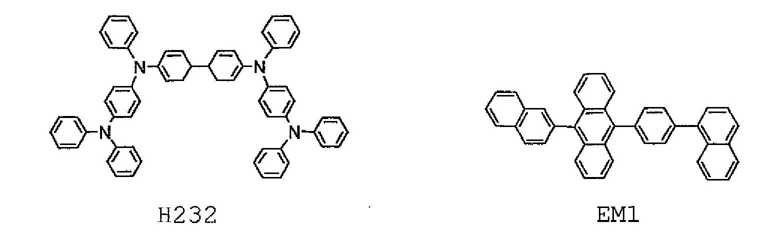

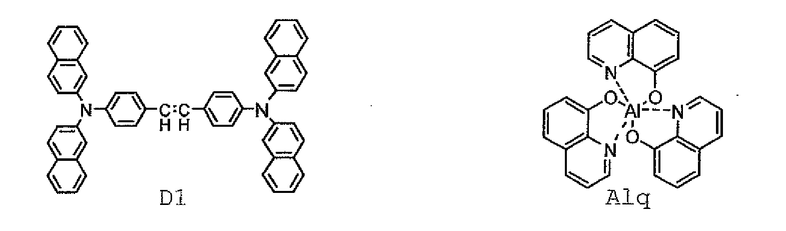

### Examples 2 to 11 (production of organic EL devices)

Organic EL devices were each produced in the same manner as in Example 1 except that any one of the compounds shown in Table 1 was used as a hole transporting material instead of Compound H1.
The current efficiency of each of the resultant organic EL devices was measured, and the luminescent color of each of the devices was observed. Further, the half lifetime of light emission in DC constant current driving at an initial luminance of 5, 000 cd/m² and room temperature was measured. Table 1 shows the results thereof.

### Comparative Example 1

An organic EL device was produced in the same manner as in Example 1 except that Comparative Compound 1 was used as a hole transporting material instead of Compound H1 (Comparative Example 1). Comparative Compound 1 crystallized at the time of vapor deposition, so a proper device could not be produced.
In addition, the current efficiency of the resultant organic EL device was measured, and the luminescent color of the device was observed. Further, the half lifetime of light emission in DC constant current driving at an initial luminance of 5,000 cd/m² and room temperature was measured. Table 1 shows the results thereof.

### Comparative Example 2 (production of organic EL devices)

An organic EL device was produced in the same manner as in Example 1 except that Comparative Compound 2 was used as a hole transporting material instead of Compound H1.
The current efficiency of the resultant organic EL device was measured, and the luminescent color of the device was observed. Further, the half lifetime of light emission in DC constant current driving at an initial luminance of 5,000 cd/m² and room temperature was measured. Table 1 shows the results thereof.

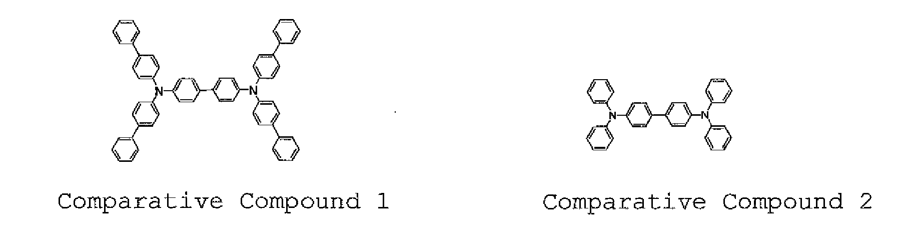

**Table 1**

| Example | Hole transporting material | Current efficiency (cd/A) | Luminescent color | Half lifetime (h) |
|---|---|---|---|---|
| 1 | H1 | 5.1 | Blue | 440 |
| 2 | H2 | 5.1 | Blue | 420 |
| 3 | H3 | 4.9 | Blue | 410 |
| 4 | H4 | 5 | Blue | 450 |
| 5 | H5 | 5.1 | Blue | 410 |
| 6 | H6 | 5 | Blue | 420 |
| 7 | H7 | 4.8 | Blue | 400 |
| 8 | H8 | 4.9 | Blue | 360 |
| 9 | H9 | 4.8 | Blue | 370 |
| 10 | H10 | 5.3 | Blue | 460 |
| 11 | H11 | 5.4 | Blue | 440 |
| Comparative Example 1 | Comparative Compound 1 | 5.1 | Blue | 250 |
| Comparative Example 2 | Comparative Compound 2 | 4.9 | Blue | 150 |

### Example 13 (production of organic EL device)

An organic EL device was produced in the same manner as in Example 1 except that Arylamine Compound D2 to be described below was used instead of Amine Compound D1 having a styryl group. Me represents a methyl group.
The measured current efficiency of the resultant organic EL device was 5.2 cd/A, and the luminescent color of the device was blue. Further, the half lifetime of light emission in DC constant current driving at an initial luminance of 5,000 cd/m² and room temperature was measured. The measured half lifetime was 430 hours.

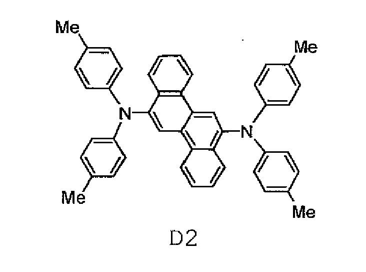

### Comparative Example 3

An organic EL device was produced in the same manner as in Example 13 except that Comparative Compound 1 described above was used instead of Compound H1 as a hole transporting material.
The measured current efficiency of the resultant organic EL device was 4.9 cd/A, and the luminescent color of the device was blue. Further, the half lifetime of light emission in DC constant current driving at an initial luminance of 5,000 cd/m² and room temperature was measured. The measured half lifetime was 260 hours.

### INDUSTRIAL APPLICABILITY

As described above in detail, an interaction between molecules in the aromatic amine derivative of the present invention is small because the derivative has steric hindrance. Accordingly, crystallization is suppressed, yield in which an organic EL device is produced is improved, and, further, the derivative can be deposited from the vapor at a low sublimation temperature. As a result, the decomposition of a molecule at the time of vapor deposition is suppressed, and an organic EL device having a long lifetime can be obtained.

## Claims

1. An aromatic amine derivative represented by the following general formula (1): 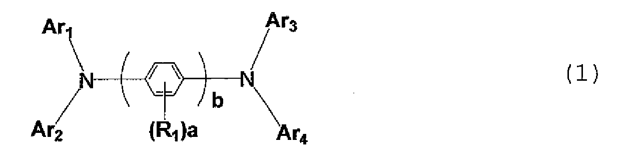 where:
R₁ represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstitutedaryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxyl group, or a carboxyl group;
a represents an integer of 0 to 4, b represents an integer of 1 to 3, and, when b represents 2 or more, multiple R₁s may be bonded to each other to forma saturated or unsaturated, five-membered or six-membered cyclic structure which may be substituted;
at least one of Ar₁ to Ar₄ represents a group of the following general formula (2): 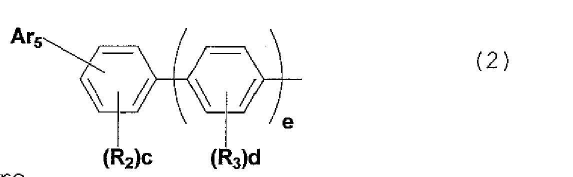 where
R₂ and R₃ are each independently selected from the same groups as those of R₁ in the general formula (1),
Ar₅ represents a fused aromatic ring group having 6 to 20 ring carbon atoms,
c and d each represent an integer of 0 to 4, and e represents an integer of 0 to 2, and
R₂ and R₃, or multiple R₃s may be bonded to each other to form a saturated or unsaturated, five-membered or six-membered cyclic structure which may be substituted; and
groups among Ar₁ to Ar₄, represented by the general formula (2) each independently represent a substituted or unsubstituted aryl group having 6 to 50 ring carbon atoms, or a substituted or unsubstituted aromatic heterocyclic group having 5 to 50 ring carbon atoms.

2. An aromatic amine derivative according to claim 1, wherein Ar₁ and Ar₂ in the general formula (1) are each represented by the general formula (2).

3. An aromatic amine derivative according to claim 1, wherein Ar₁ and Ar₃ in the general formula (1) are each represented by the general formula (2).

4. An aromatic amine derivative according to any one of claims 1 to 3, wherein e in the general formula (2) represents 0.

5. An aromatic amine derivative according to any one of claims 1 to 4, wherein Ar₅ in the general formula (2) represents a 1-naphthyl group, a 2-naphthyl group, a phenanthryl group, or a pyrenyl group.

6. An aromatic amine derivative according to claim 1, wherein Ar₂ in the general formula (1) is represented by the following general formula (3): 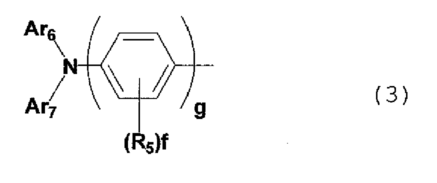 where:
R₅ is selected from the same groups as those of R₁ in the general formula (1);
f represents an integer of 0 to 4, and g represents an integer of 1 to 3;
when g represents 2 or more, multiple R₅s may be bonded to each other to form a saturated or unsaturated, five-membered or six-membered cyclic structure which may be substituted; and
Ar₆ and Ar₇ are each independently represented by the general formula (2), or each independently represent a substituted or unsubstituted aryl group having 6 to 50 ring carbon atoms, or a substituted or unsubstituted aromatic heterocyclic group having 5 to 50 ring carbon atoms.

7. An aromatic amine derivative according to claim 1, wherein Ar₂ and Ar₄ in the general formula (1) are each represented by the general formula (3).

8. An aromatic amine derivative according to any one of claims 1 to 7, which is used as a material for an organic electroluminescence device.

9. An aromatic amine derivative according to any one of claims 1 to 7, which is used as a hole transporting material for an organic electroluminescence device.

10. An organic electroluminescence device comprising one or multiple organic thin film layers including at least a light emitting layer, the one or multiple organic thin film layers being interposed between a cathode and an anode,
wherein at least one layer of the one or more multiple organic thin film layers contains the aromatic amine derivative according to any one of claims 1 to 7 alone or as a component of a mixture.

11. An organic electroluminescence device according to claim 10, wherein:
the organic thin film layers have a hole transporting layer; and
the hole transporting layer contains the aromatic amine derivative alone or as a component of a mixture.

12. An organic electroluminescence device according to claim 10 or 11, wherein the organic electroluminescence device emits bluish light.

13. An organic electroluminescence device according to claim 12, wherein the light emitting layer contains styrylamine and/or arylamine.
